# EUROPEAN PATENT APPLICATION

(11) **EP 4 344 624 A1**
(43) Date of publication of application: **03.04.2024**
(21) Application number: 21948567.9
(22) Date of filing: 10.11.2021
(51) Int. Cl.: A61B 5/145, A61B 5/155, A61B 5/15, A61M 5/172, A61M 5/142

(54) **APPLICATOR FOR TRANSCUTANEOUS SENSOR, AND APPLICATOR ASSEMBLY**

(30) Priority: 29.06.2021 KR 20210084622
(71) Applicant: i-Sens, Inc., Seoul 06646 (KR)
(72) Inventor: CHOI, Hyun Ho, Seoul 06646 (KR); RYU, Goang Yel, Seoul 06646 (KR); WANG, Ji Hoon, Seoul 06646 (KR)
(74) Representative: BCKIP Part mbB
(86) International application number: PCT/KR2021/016303
(87) International publication number: WO 2023/277267

(57) **Abstract**

An applicator, according to the present invention, for inserting, into the skin of a user, a sensor for measuring biological information comprises: an applicator body, which can come in contact with the skin of a user; an insertion unit provided at the applicator body in order to move, from a first position at which a sensor unit is spaced from the skin of the user to a second position at which the sensor unit is inserted into the skin of the user, the sensor unit, including the sensor, a sensor unit housing in which the sensor is mounted, an adhesive layer provided on the sensor unit housing, and a protective sheet for covering the adhesive layer; a removing unit provided at the applicator body so as to separate the protective sheet from the adhesive layer before the sensor unit reaches the second position; and an operating member provided at the applicator body so as to be operated by the user, and thus enable the removing unit to be operated.

## Description

### TECHNICAL FIELD

The present disclosure relates to an applicator for a transcutaneous sensor, and more specifically, related to an applicator and an applicator assembly for inserting a transcutaneous sensor, which is inserted into the skin of a user to measure biometric information, into the skin of a user.

### BACKGROUND

With the recent advancement of medical technology, various medical devices that are attached to the body of a user have been developed and sold. Medical devices that are attached to the skin can be useful for monitoring biometric information or providing treatment by attaching them to the body of a patient with a chronic disease.

For example, chronic diseases such as diabetes require continuous management, and a body attachable unit for measuring biometric information can be used to manage blood glucose levels in diabetic patients. Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown. Further progression of diabetes leads to complications such as visual disturbances, hypertension, kidney disease, paralysis, periodontal disease, muscle spasms and neuralgia, as well as gangrene. In order to diagnose diabetes beforehand and manage to prevent the progression of diabetes into complications associated therewith, systematic blood glucose measurement and treatment should be performed.

For diabetes patients as well as people having higher than normal blood glucose, even though diabetes has not yet developed, medical device manufacturers offer a variety of blood glucose meters to measure blood glucose levels.

Glucose measuring devices may be categorized into a single time measurement type measuring a blood glucose level and collecting blood from a fingertip by a user every single time and a continuous measurement type attaching a glucose monitoring system to the belly or an arm of the user and continuously measuring blood glucose levels.

Diabetics patients generally experience hyperglycemia and hypoglycemia, an emergency may occur in the hypoglycemic conditions, and the patients may become unconscious or die if a hypoglycemic condition lasts for an extended period of time without the supply of sugar. Accordingly, although rapid discovery of the hypoglycemic condition is critically important for diabetics, blood-collecting type glucose monitoring devices intermittently measuring glucose have limited ability to accurately measure blood glucose levels.

Recently, to overcome such a drawback, continuous glucose monitoring systems (CGMSs) inserted into the human body to measure a blood glucose level every few minutes have been developed, and therefore easily perform the management of diabetics and responses to an emergency situation.

A continuous blood glucose measurement system includes a body attachable unit that includes a transcutaneous sensor that is inserted into the skin of a user and measures blood glucose levels in the body, and a terminal that receives biometric information transmitted from the body attachable unit and outputs.

Systems for medical purposes using transcutaneous sensors are produced in many different forms by each manufacturer, and the methods of use also vary. Most systems currently manufactured and distributed are a method of attaching a disposable sensor unit to a body by an applicator. A user needs to perform several steps to attach the sensor unit to a skin using an applicator, and after attaching the sensor unit to a body, various follow-up procedures such as having to directly pull out the needle inserted into the skin along with the transcutaneous sensor are required to be performed.

For example, a user must peel off the packaging of the disposable sensor unit and accurately attach it to the applicator, and then insert the sensor unit into the applicator and operate the applicator to attach the sensor unit to a skin. Additionally, after attaching the sensor unit, there is the inconvenience of having to perform tasks such as pulling out the needle inserted into the skin and coupling the transmitter to the sensor unit.

### SUMMARY

### Technical Problem

The present disclosure is developed in consideration of the above-mentioned points, and the purpose of the present disclosure is to minimize the additional work to insert the transcutaneous sensor into a skin by enabling the sensor unit including the transcutaneous sensor to be manufactured in an assembled state, and to provide an applicator and applicator assembly for a transcutaneous sensor that can be conveniently used by a user to insert a transcutaneous sensor into the skin.

Another purpose of the present disclosure is to provide an applicator for a transcutaneous sensor and applicator assembly that can automatically separate the protective sheet that covers and protects the adhesive layer provided on the sensor unit.

### Solution to Problem

To accomplish the above-described purposes, an applicator for inserting a sensor for measuring biometric information into skin of a user according to an embodiment of the present disclosure may comprise: an applicator body configured to be contactable with the skin of the user; an insertion unit installed to the applicator body to move a sensor unit, which comprises the sensor, a sensor unit housing to which the sensor is mounted, an adhesive layer comprised in the sensor unit housing, and a protective sheet covering the adhesive layer, from a first position where the sensor unit is spaced apart from the skin of the user to a second position where the sensor is inserted into the skin of the user; a removing unit installed to the applicator body to separate the protective sheet from the adhesive layer before the sensor unit reaches the second position; and an operating member installed to the applicator body such that the removing unit is operated by manipulation of the user.

The removing unit may comprise a moving member installed movably to the applicator body to pull the protective sheet in a direction of separating the protective sheet from the adhesive layer, wherein a portion of the protective sheet is coupled to the moving member.

The removing unit may include a moving member driver configured to move the moving member.

The moving member driver may include an elastic member configured to apply an elastic force to the moving member in a direction of peeling off the protective sheet from the adhesive layer.

The applicator according to an embodiment of the present disclosure may comprise a stopper configured to restrain movement of the moving member in a state that the elastic member is elastically deformed, wherein the operating member is configured to release the stopper such that the moving member is movable by the elastic member.

The moving member may be installed to move in a direction of intersecting a direction in which the sensor unit moves from the first position to the second position.

The applicator according to an embodiment of the present disclosure may comprise a support installed to the applicator body to support the protective sheet separated from the adhesive layer by the moving member.

The moving member may be installed to be movable from a third position where the protective sheet is coupled to the protective sheet in a state that the protective sheet covers the adhesive layer to a fourth position where the protective sheet is separated from the adhesive layer, and the support may be configured to be elastically deformable by being pressed by the moving member when the moving member is in the third position, and return to an original state to support the protective sheet by being released from the moving member when the moving member moves to the fourth position.

The insertion unit may include a plunger installed to be movably from the first position to the second position with the sensor unit, a needle detachably coupled to the sensor unit to be inserted into the skin of the user with the sensor, a carrier installed to the plunger and coupled with the needle, and a plunger driver configured to provide a moving force to the plunger in a direction of moving from the first position to the second position, and the plunger may be configured such that movement of the plunger at the first position is restricted by being contacted with the moving member, and is configured to move away from the moving member toward the second position when the moving member moves in a direction of separating the protective sheet from the adhesive layer.

The moving member may be installed to move in a direction of intersecting a direction in which the sensor unit moves from the first position to the second position, and the plunger may be configured to deviate from the moving member after a separation operation of the protective film by the moving member is completed.

The applicator body may include a frame base portion configured to be contactable with the skin of the user, a base frame having a column portion protruding from the frame base portion and accommodating the plunger, a stage disposed at an upper side of the frame base and supporting the moving member, and a middle frame having a middle frame opening formed in a middle of the stage to allow the column portion to be inserted.

The removing unit may comprise an elastic member configured to apply an elastic force to the moving member in a direction of removing the protective sheet from the adhesive layer, a stopper configured to restrains movement of the moving member in a state in which the elastic member is elastically deformed is comprised in one of the middle frame and the moving member, and the operating member is configured to release the stopper such that the moving member is movable by the elastic member.

The moving member may comprise a moving member body, a moving member opening formed in a middle of the moving member body to allow the plunger to pass through the moving member opening, and a grip portion comprised in the moving member body to be coupled with the protective sheet.

The applicator according to an embodiment of the present disclosure may further comprise a needle release driver configured to provide a moving force to the carrier such that the carrier moves the needle in a direction away from the skin of the user after the needle is inserted into the skin of the user with the sensor.

The protective sheet may comprise a protective portion covering the adhesive layer, and a wing portion extending from an edge of the protective portion to be coupled to the moving member.

The protective sheet may have a protective sheet groove, and the moving member may include a grip portion inserted into the protective sheet groove.

Additionally, to accomplish the above-described purposes, an applicator for inserting a sensor into skin of a user according to another embodiment of the present disclosure may comprise: an applicator body to which a base unit comprising a base unit housing and an adhesive portion provided on the base unit housing to be attached to the skin of the user is separatably coupled; an insertion unit installed to the applicator body to move the sensor unit, which comprises the sensor, a sensor unit housing to which the sensor is mounted, an adhesive layer comprised in the sensor unit housing to be adhered to the base unit housing, and a protective sheet covering the adhesive layer, from a first position where the sensor unit is spaced apart from the base unit to a second position where the sensor unit is coupled to the base unit such that the sensor is inserted into the skin of the user; a removing unit installed to the applicator body to separate the protective sheet from the adhesive layer before the sensor unit reaches the second position; and an operating member installed to the applicator body such that the removing unit is operated by manipulation of the user.

Additionally, to accomplish the above-described purposes, an applicator assembly according to an embodiment of the present disclosure may comprise: a sensor unit comprising a sensor, a sensor unit housing to which the sensor is mounted, an adhesive layer comprised in the sensor unit housing, and a protective sheet covering the adhesive layer; an applicator body having a bottom portion configured to be contactable with skin of an user; an insertion unit installed to the applicator body to move the sensor unit from a first position where the sensor unit is spaced apart from the skin of the user to a second position where the sensor unit is inserted into the skin of the user; a removing unit installed to the applicator body to separate the protective sheet from the adhesive layer before the sensor unit reaches the second position; and an operating member installed to the applicator body such that the removing unit is operated by manipulation of the user.

The applicator assembly according to an embodiment of the present disclosure may further comprise a base unit comprising a base unit housing and an adhesive portion provided on the base unit housing to be attached to the skin of the user, wherein the base unit separatably coupled to the applicator body, wherein the sensor unit housing is attached to the base unit housing by the adhesive layer at the second position.

### Advantageous Effects of Invention

According to the present disclosure, by manufacturing the sensor unit in an assembled state within the applicator, the additional work for a user to attach the sensor unit to the skin of a user is minimized, and the sensor unit can be attached to the skin of a user simply by operating the applicator.

Additionally, according to the present disclosure, by covering and protecting the adhesive layer of the sensor unit with a protective sheet, it may prevent the problem of deterioration of the adhesiveness of the adhesive layer before inserting the sensor into the skin of a user.

In addition, according to the present disclosure, the protective sheet that covers and protects the adhesive layer of the sensor unit can be automatically separated by operating the applicator.

### BRIEF DESCRIPTION OF THE DRAWINGS

FIG. 1 is a perspective view showing an applicator assembly according to an embodiment of the present disclosure.
FIG. 2 shows a body attachable unit attached to the skin of a user.
FIG. 3 is a perspective view showing a body attachable unit.
FIGS. 4 and 5 are exploded perspective views showing a sensor unit.
FIG. 6 is a cross-sectional view showing a portion of a sensor unit disassembled.
FIG. 7 is a cross-sectional view showing a needle coupled to a sensor unit.
FIG. 8 is a cross-sectional view showing a needle being separated from a sensor unit.
FIG. 9 shows a protective sheet attached to an adhesive layer of a sensor unit.
FIG. 10 shows the appearance after the protective sheet is separated from an adhesive layer of a sensor unit.
FIGS. 11 and 12 show a sensor unit and a base unit before they are coupled.
FIG. 13 is a cross-sectional view showing a body attachable unit assembled by coupling a sensor unit and a base unit.
FIG. 14 is a cross-sectional view taken along line I-I in FIG. 1.
FIG. 15 is a cross-sectional view taken along line II-II in FIG. 1.
FIG. 16 is exploded perspective views of an applicator according to an embodiment of the present disclosure.
FIGS. 17 and 18 are exploded perspective views showing a needle assembly of an applicator according to an embodiment of the present disclosure.
FIG. 19 is a cross-sectional view showing a carrier and a needle before being coupled
FIG. 20 is a perspective view showing a partial configuration of an applicator according to an embodiment of the present disclosure.
FIGS. 21 and 22 show the moving member, sensor unit, and carrier of the applicator according to an embodiment of the present disclosure being separated.
FIG. 23 shows a view in which the operating member of the applicator according to an embodiment of the present disclosure biases the stopper to be disengaged from the moving member.
FIG. 24 is a side view showing the moving member of the applicator according to an embodiment of the present disclosure fixed in the third position by a stopper.
FIG. 25 is a plan view showing the moving member of the applicator according to an embodiment of the present disclosure fixed in the third position by a stopper.
FIG. 26 is a side view showing the moving member according to an embodiment of the present disclosure moving to the fourth position and removing the protective sheet of the sensor unit.
FIG. 27 is a plan view showing the moving member of the applicator according to an embodiment of the present disclosure moved to the fourth position
FIG. 28 shows the locking hook of the applicator according to an embodiment of the present disclosure engaged with the base unit.
FIGS. 29 and 30 show the locking hook of the applicator according to an embodiment of the present disclosure connected to the guide portion of the moving member.
FIGS. 31 to 36 show a process in which the sensor of the sensor unit is inserted into the skin of a user by an applicator according to an embodiment of the present disclosure.
FIGS. 37 and 38 are cross-sectional views showing an applicator assembly according to another embodiment of the present disclosure.
FIG. 39 shows the top case of the applicator assembly according to an embodiment of another disclosure separated from the middle frame.
FIG. 40 is a cross-sectional view showing a portion of an applicator assembly according to another embodiment of the present disclosure.
FIGS. 41 and 42 show the moving member, sensor unit, and carrier of the applicator according to another embodiment of the present disclosure being separated.
FIGS. 43 and 44 show a process in which the moving member of the applicator, according to another embodiment of the present disclosure, separates the protective sheet of the sensor unit.
FIG. 45 is a cross-sectional view showing a portion of an applicator according to another embodiment of the present disclosure.
FIG. 46 shows the moving member of the applicator according to another embodiment of the present disclosure fixed in the third position by a stopper.
FIG. 47 shows a state in which the moving member of the applicator moves to the fourth position and separates the protective sheet of the sensor unit according to another embodiment of the present disclosure.
FIG. 48 is a cross-sectional view showing a portion of an applicator assembly according to another embodiment of the present disclosure.
FIG. 49 shows the sensor unit of the applicator assembly shown in FIG. 48.
FIGS. 50 and 51 show another embodiment of the sensor unit and base unit.

### DETAILED DESCRIPTION OF EMBODIMENTS

Hereinafter, the applicator and applicator assembly for a transcutaneous sensor according to the present disclosure will be described in detail with reference to the drawings.

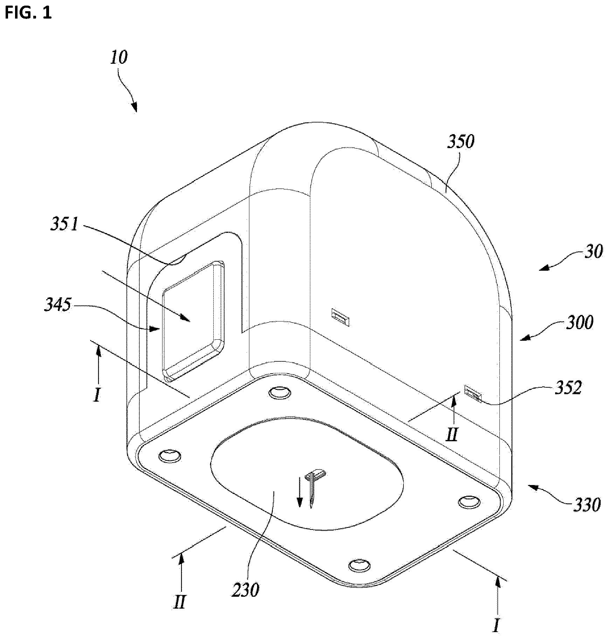

FIG. 1 is a perspective view showing an applicator assembly according to an embodiment of the present disclosure.

An applicator assembly (10) according to an embodiment of the present disclosure includes a sensor unit (100) including a sensor (110) that is inserted into the skin of a user and measures biometric information, and a base unit (200) that is coupled with the sensor unit (100) to form a body attachable unit (20), and an applicator (30) for attaching the body attachable unit (20) to the skin of a user. The applicator assembly (10) may be provided to a user with the sensor unit (100) and the base unit (200) mounted separately on the applicator (30). The sensor unit (100) and the base unit (200) can be automatically assembled and attached to the skin of a user by the operation of the applicator (30).

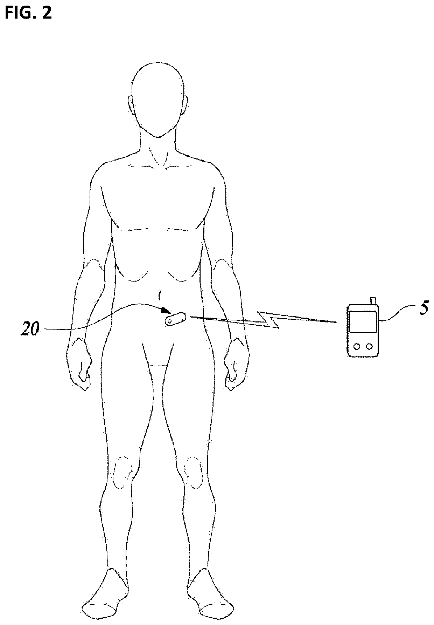

As shown in FIG. 2, the body attachable unit (20) can be attached to the skin of a user by the applicator (30), measure biometric information, and wirelessly transmit the measured data to an external terminal (5). The external terminal (5) may be a variety of devices capable of receiving measurement data from the body attachable unit (20), such as a portable terminal, a medical device, a PC, or a server. Biometric information that the body attachable unit (20) can measure is not limited to specific information. As an exemplary embodiment, the body attachable unit (20) may periodically measure blood glucose level for a user and transmit the blood glucose measurement information to the external terminal (5).

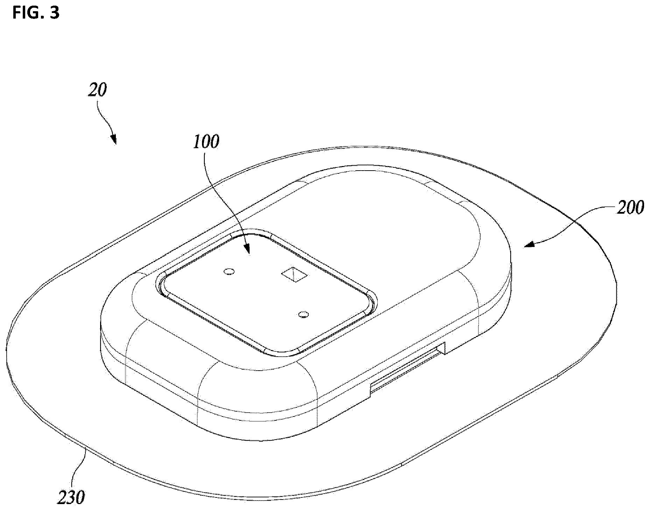

As shown in FIG. 3, the body attachable unit (20) includes the sensor unit (100) for measuring the biometric information of a user, and the base unit (200) that is attached to the skin of a user and coupled to the sensor unit (100). do. The base unit (200) may have electronic components installed therein. This base unit (200) is electrically connected to the sensor unit (100) and can process biometric information measured by the sensor unit (100) and transmit it to the external terminal (5).

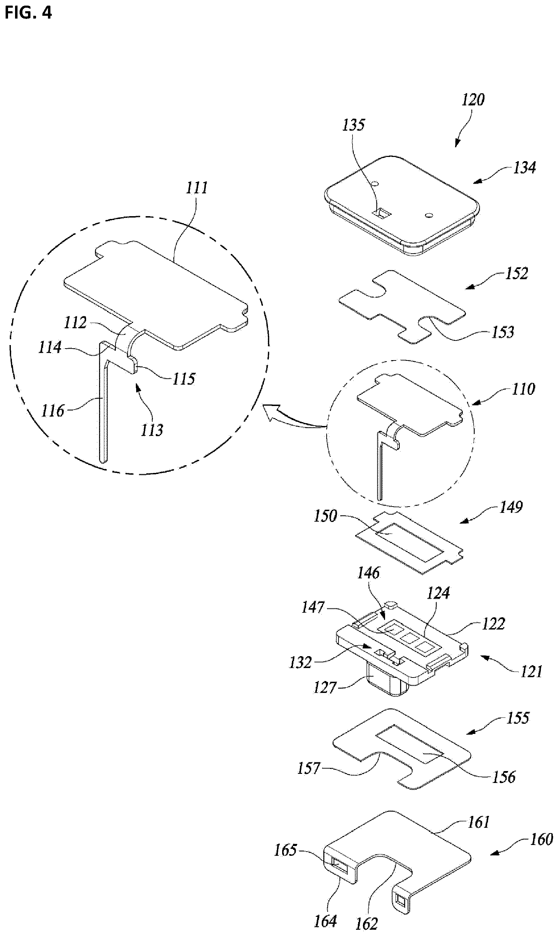

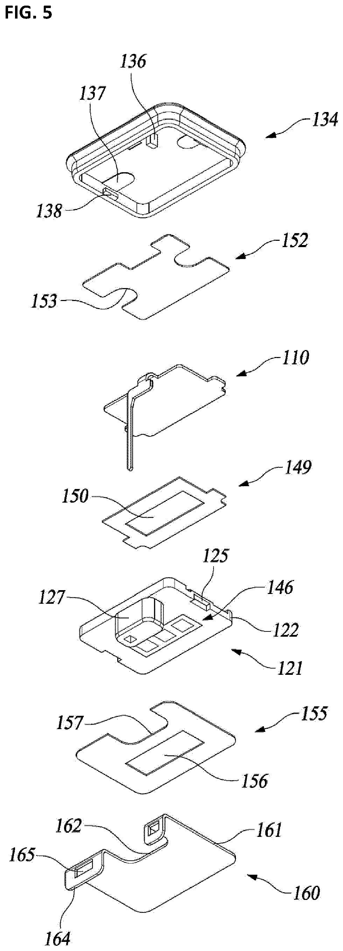

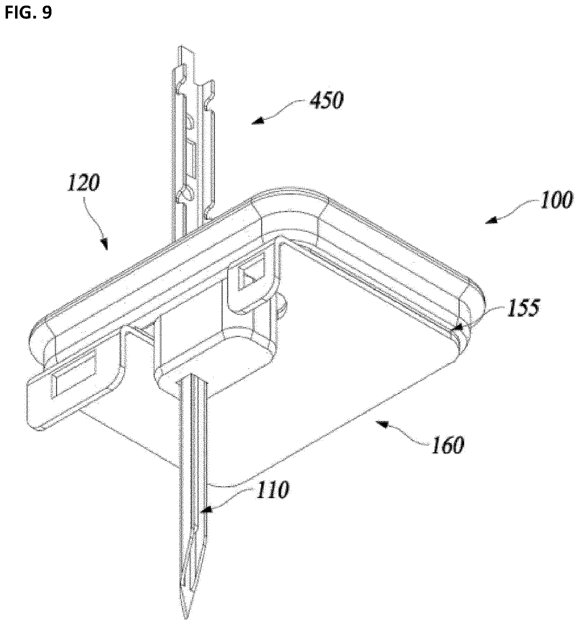

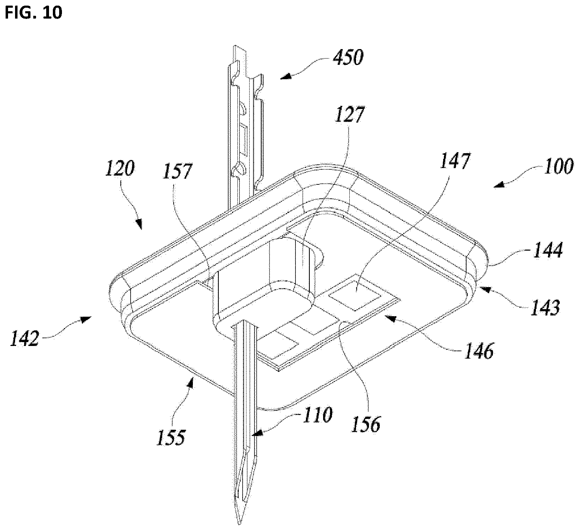

As shown in FIGS. 4 to 10, the sensor unit (100) includes the sensor (110) inserted into the skin of a user, a sensor unit housing (120) to which the sensor (110) is coupled, a sensor unit-electrical contact portion(146) electrically connected to the sensor (110) coupling to the sensor unit housing(120), a sensor adhesive portion (149) and an adhesive pad (152) for fixing the sensor (110) to the sensor unit housing (120), an adhesive layer (155) provided on the sensor unit housing (120) so as to be attached to the base unit (200), a protective sheet (160) covering the adhesive layer (155).

The sensor (110) includes a sensor body (111), a connection portion (112) connected to one side of the sensor body (111), a middle portion (113) connected to the sensor body (111) through the connection portion (112), and an insertion portion (116) connected to the middle portion (113) so as to be inserted into the skin of a user. The sensor body (111) is disposed inside the sensor unit housing (120) so as to be in contact with the sensor unit-electrical contact portion (146). The sensor body (111) may be provided with an electrode electrically connected to the sensor unit-electrical contact portion (146). The connection portion (112) extends from the edge of the sensor body (111) and connects the sensor body (111) and the middle portion (113) to lie on different planes. The sensor (110) may be manufactured in a flat form in which the sensor body (111) lies on the same plane as the middle portion (113) and the insertion portion (116), and then manufactured in a form in which the connection portion (112) is bent and deformed so that the sensor body (111) lies on a different plane from the middle portion (113) and the insertion portion (116).

The middle portion (113) and the insertion portion (116) are connected to the sensor body (111) so as to lie on a different plane from the sensor body (111). In the drawing, the middle portion (113) and the insertion portion (116) are shown to be disposed on the same plane, and the middle portion (113) is disposed perpendicular to the sensor body (111), but the angle between the middle portion (113) and the sensor body (111) or the angle between the insertion portion (116) and the sensor body (111) can be changed in various ways.

The middle portion (113) includes a first extension portion (114) that protrudes to one side of the connection portion (112), and a second extension portion (115) that protrudes from the connection portion (112) in a direction opposite to the direction in which the first extension portion (114) protrudes from the connection portion (112). The insertion portion (116) may be connected to the first extension portion (114) and inserted into the skin of a user. The insertion portion (116) has a relatively thin and long shape so as to be smoothly inserted into the skin of a user. The connection portion (112), the middle portion (113), and the insertion portion (116) may be provided with a conductive trace electrically connected to the electrode of the sensor body (111). The sensor (110) may be coupled to the sensor unit housing (120) so that the sensor body (111), the connection portion (112) and the middle portion (113) are located inside the sensor unit housing (120) and only the insertion portion (116) protrudes from the sensor unit housing (120).

The sensor unit housing (120) includes a housing base (121) and a housing cap (134) that covers the top of the housing base (121). A space is provided inside the sensor unit housing (120) to accommodate the sensor (110), and a housing opening (140) into which a needle (450) for inserting the sensor (110) into the skin of a user is inserted is formed in the middle of the sensor unit housing (120) to penetrate the sensor unit housing (120) in the thickness direction.

The housing base (121) includes a base portion (122) supporting the sensor body (111) of the sensor (110), and a boss (127) protruding from the surface of the base portion (122). On one side of the base portion (122), a base portion hole (123) is formed to penetrate the base portion (122), and on the other side of the base portion (122), a through hole (124) is formed to penetrate the base portion (122). A sensor unit-electrical contact portion (146) is coupled to the through hole (124). A detent groove (125) is provided at the edge of the base portion (122).

The boss (127) protrudes from the surface on the side of the base portion (122) on which the sensor (110) is not placed. A boss hole (128) is formed in the boss (127) to penetrate the boss (127). The boss hole (128) is connected to the base portion hole (123) and forms a housing base hole (132) together with the base hole (123). The boss hole (128) includes an upper hole (129) connected to the base portion hole (123) and a lower hole (130) connected to the upper hole (129) and extending to the end of the boss (127). The size of the upper hole (129) is larger than the size of the lower hole (130). The upper hole (129) is sized to accommodate the middle portion (113) of the sensor (110) and portion of the needle (450), and the lower hole (130) is sized to accommodate the insertion portion (116) of the sensor (110) and a portion of the needle (450). The upper hole (129) is provided with a support portion (131) capable of supporting the middle portion (113) of the sensor (110). As the support portion (131) supports the middle portion (113), the sensor (110) does not move while coupled to the sensor unit housing (120) and can maintain a stable coupling state with the sensor unit housing (120).

The housing cap (134) is coupled to the housing base (121) and covers the sensor body (111) of the sensor (110) placed on the housing base (121) and the upper part of the housing base (121). A housing cap hole (135) is formed in the middle of the housing cap (134) to penetrate the housing cap (134) in the thickness direction. The housing cap hole (135) is connected to the housing base hole (132) of the housing base (121) to form the housing opening (140). The needle (450) for inserting the sensor (110) into the skin of a user is inserted along with the sensor (110) into the housing opening (140). The housing cap (134) is provided with a detent protrusion (138) corresponding to the detent groove (125) of the housing base (121). The housing cap (134) may be coupled to the housing base (121) by inserting the detent protrusion (138) into the detent groove (125) and engaging the housing base (121). A protrusion (137) and a retention protrusion (136) are provided on the inner surface of the housing cap (134) facing the housing base (121). The protrusion (137) and the retention protrusion (136) protrude toward the housing base (121). The protrusion (137) and the retention protrusion (136) may contact the sensor (110) to prevent movement of the sensor (110) and stably fix the sensor (110) to the sensor unit housing (120). The protrusion (137) may contact the sensor body (111) of the sensor (110) and press the sensor body (111) toward the housing base (121). Therefore, the sensor body (111) can remain stably fixed to the housing base (121) without being lifted from the housing base (121).

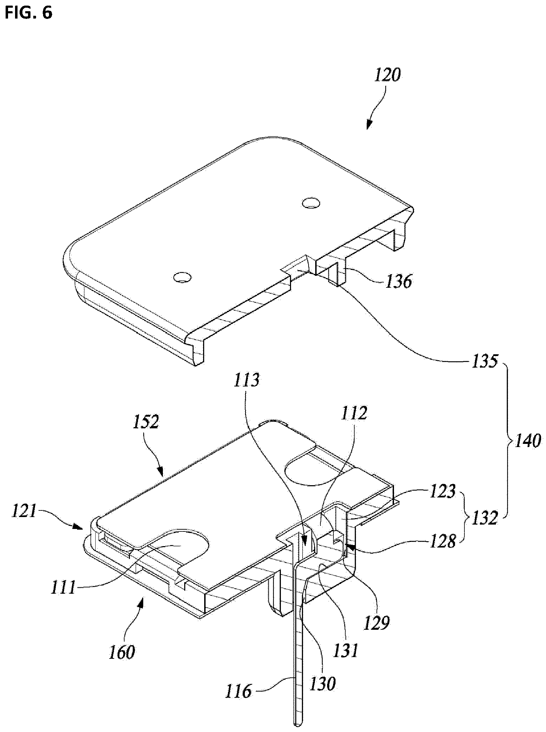

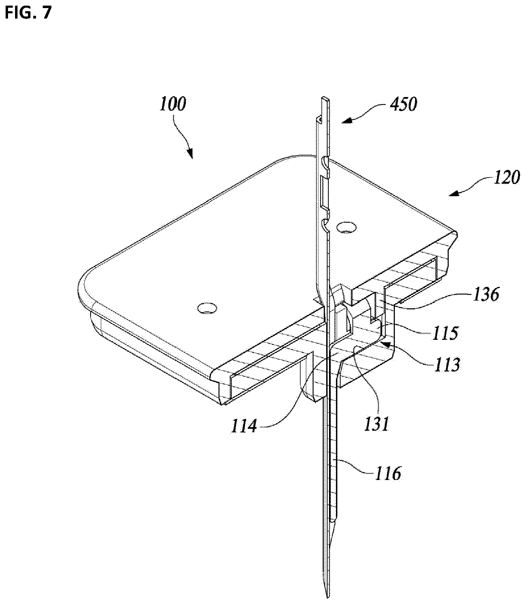

As shown in Figures 6 and 7, when the housing cap (134) is coupled to the housing base (121), the retention protrusion (136) is inserted into the housing base hole (132) and comes into contact with the sensor (110). That is, the retention protrusion (136) is arranged so that its end faces the second extension (115) of the middle portion (113) inserted into the housing base hole (132), and may restrain the movement of the middle portion (113) of the sensor (110) so that the middle portion (113) does not lift off the support portion (131). Due to the action of these retention protrusions (136), the insertion portion (116) of the sensor (110) can be stably maintained in a state of protruding from the sensor unit housing (120) by a certain length. And, due to the action of the retention protrusion (136), the insertion portion (116) of the sensor (110) does not retreat from the skin of a user while being inserted into the skin of a user, and can remain inserted at a certain depth into the skin of a user.

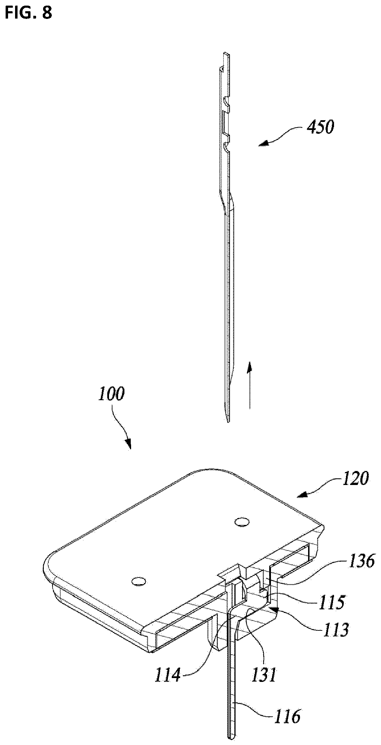

Also, as shown in FIG. 8, after the insertion portion (116) is inserted into the skin of a user together with the needle (450), the retention protrusion (136) prevents the middle portion (113) from moving during the process of the needle (450) coming out of the skin of a user, so that the insertion portion (116) cannot retract in the same direction as the needle (450). Accordingly, it is possible to prevent a problem in which the insertion portion (116) inserted into the skin of a user moves in the direction in which the needle (450) exits the skin of a user during the process of the needle (450) coming out of the skin of a user. In addition, it may prevent the problem of the insertion portion (116) partially coming out of the skin and reducing the measurement accuracy of the sensor (110).

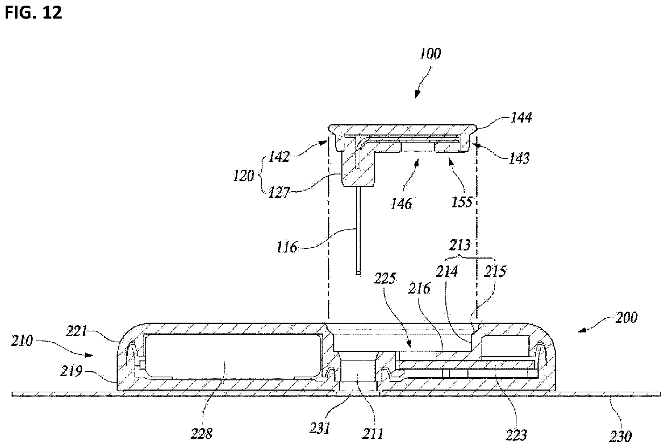

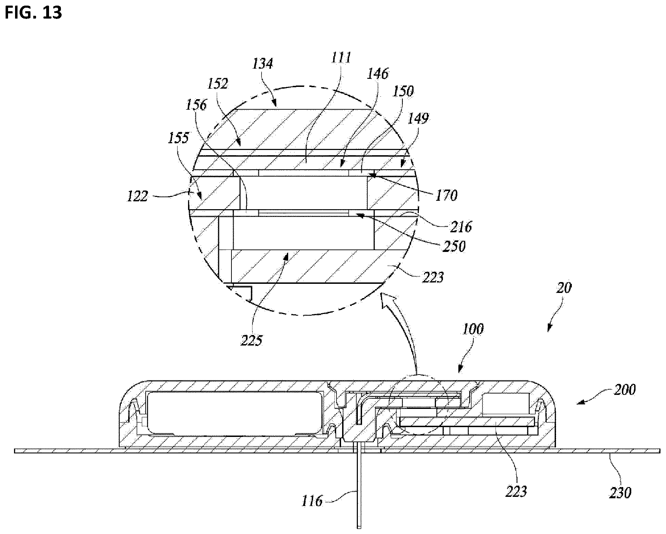

The housing cap (134) forms the housing body (142) together with the base portion (122). That is, the sensor unit housing (120) may be configured to include a housing body (142) and a boss (127) protruding from one side of the housing body (142). As shown in FIGS. 12 and 13, the housing body (142) is shaped to fit into a base unit recess (213) of the base unit (200). The housing body (142) includes a body portion (143) provided with a boss (127) and a cover portion (144) that is wider than the body portion (143).

The sensor unit housing (120) is not limited to the configuration shown, and may be changed to various other configurations on which the sensor (110) may be mounted and coupled to the base unit (200). That is, the sensor unit housing (120) includes a housing base (121) and the housing cap (134), but it can be changed to various configurations other than a configuration including a housing body (142) and a boss (127) protruding from the housing body (142).

The sensor unit-electrical contact portion (146) is disposed in the sensor unit housing (120) to be electrically connected to the sensor body (111) of the sensor (110). The sensor unit-electrical contact portion (146) is inserted into the through hole (124) of the base portion (122) and is disposed to vertically penetrate the base portion (122). The sensor unit-electrical contact portion (146) may include a plurality of terminal portions (147) for transmitting electrical signals. Some part of the sensor unit-electrical contact portion (146) is exposed to one surface of the base portion (122) where the sensor body (111) of the sensor (110) is located, and another part is exposed to the other side of the base portion (122), so that the sensor (110) and a base unit- electrical contact portion (225) of the base unit (200) can be electrically connected.

The sensor adhesive portion (149) is disposed between the housing base (121) and the sensor body (111) of the sensor (110) to attach the sensor body (111) to the housing base (121). The sensor adhesive portion (149) has adhesive properties on both sides. That is, one side of the sensor adhesive portion (149) is adhered to the housing base (121), and the other surface of the sensor adhesive portion (149) is adhered to the sensor body (111). A sensor adhesive portion opening (150) is formed in the middle of the sensor adhesive portion (149) to penetrate the sensor adhesive portion (149) in the thickness direction. The sensor body (111) of the sensor (110) is in contact with the sensor unit-electrical contact portion (146) through the sensor adhesive portion opening (150). Therefore, the sensor adhesive portion (149) seals between the sensor body (111) and the sensor unit-electrical contact portion (146), thereby preventing moisture or foreign substances from entering an electrical connection portion (170) between the sensor body (111) and the sensor unit electrical contact portion (146).

The shape of the sensor adhesive portion (149) is not limited to that shown and can be changed in various ways.

The adhesive pad (152) is disposed between the housing cap (134) and the sensor body (111) of the sensor (110) to attach the sensor body (111) to the housing cap (134). The adhesive pad (152) has adhesive properties on both sides. That is, one side of the adhesive pad (152) is adhered to the housing cap (134), and the other side of the adhesive pad (152) is adhered to the sensor body (111). An adhesive pad groove (153) is formed in the adhesive pad (152) to penetrate the adhesive pad (152) in the thickness direction. The protrusion (137) of the housing cap (134) contacts the sensor body (111) through the adhesive pad groove (153), thereby pressing the sensor body (111) toward the housing base (121).

The shape of the adhesive pad (152) is not limited to that shown and can be changed in various ways.

An adhesive layer (155) is disposed on the surface of the housing base (121). The adhesive layer (155) has adhesive properties on both sides. One side of the adhesive layer (155) is adhered to the housing base (121), and the other side of the adhesive layer (155) is covered with the protective sheet (160). The adhesive layer (155) may be attached to the base unit (200) after the protective sheet (160) is separated. An adhesive layer hole (156) is formed in the middle portion of the adhesive layer (155) to penetrate the adhesive layer (155) in the thickness direction. The sensor unit-electrical contact portion (146) may contact the base unit-electrical contact portion (225) of the base unit (200) through the adhesive layer hole (156). Therefore, the adhesive layer (155) seals between the sensor unit-electrical contact portion (146) and the base unit-electrical contact portion (225), thereby preventing moisture or foreign substances from entering the electrical connection portion (250) between the sensor unit (100) and the base unit (200). An adhesive layer opening (157) is formed on one side of the adhesive layer (155) to penetrate the adhesive layer (155) in the thickness direction. The adhesive layer (155) is attached to the housing base (121) such that the sensor unit-electrical contact portion (146) is located inside the adhesive layer hole (156) and the boss (127) is inserted into the adhesive layer opening (157).

The shape of the adhesive layer (155) is not limited to that shown and can be changed in various ways.

The protective sheet (160) covers and protects the adhesive layer (155). The protective sheet (160) is made of a material that is detachably attached to the adhesive layer (155). If the adhesive layer (155) is left exposed to the air for a long time, the adhesiveness of the adhesive layer (155) may deteriorate. The protective sheet (160) covers the adhesive layer (155) to prevent the problem of reducing of adhesiveness of the adhesive layer (155), and it facilitates handling of the sensor unit (100) by an operator during the manufacturing process of the sensor unit (100) or the process of assembling the sensor unit (100) to the applicator (30). The protective sheet (160) includes a protection portion (161) adhered to the adhesive layer (155) and a wing portion (164) extending from an edge of the protection portion (161). A protective sheet opening (162) is formed on one side of the protection portion (161) to penetrate the protection portion (161) in the thickness direction. The protective sheet (160) is attached to the adhesive layer (155) such that the boss (127) is inserted into the protective sheet opening (162). The length of the wing portion (164) is longer than the length of the protection portion (161). A protective sheet groove (165) is formed in the wing portion (164) to penetrate the wing portion (164) in the thickness direction. The wing portion (164) may extend by a certain length from the edge of the sensor unit housing (120) and be coupled to the removing unit (500) of the applicator (30).

The shape of the protective sheet (160) is not limited to that shown and can be changed in various ways.

The sensor unit (100) is mounted on the applicator (30) with the protective sheet (160) attached to the adhesive layer (155). The protective sheet (160) may be separated from the adhesive layer (155) by a removing unit (600) of the applicator (30) before the sensor (110) is inserted into the skin of a user. The sensor unit (100) moves toward the base unit (200) with the protective sheet (160) separated and is coupled to the base unit (200).

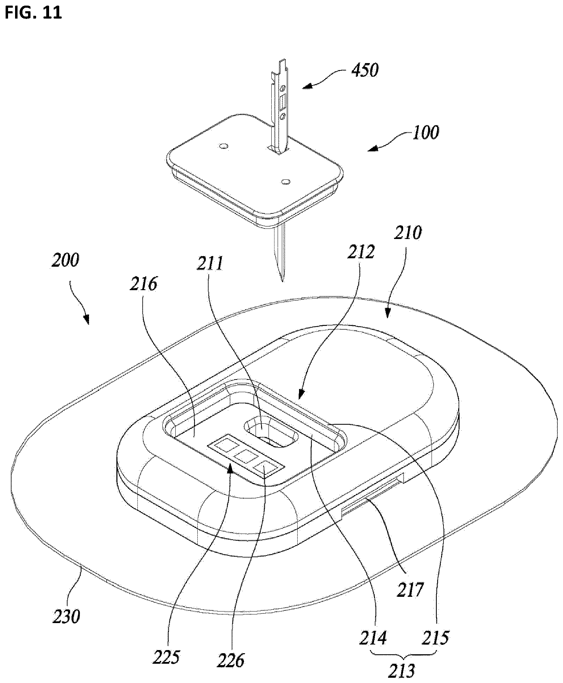

As shown in FIGS. 11 to 13, the base unit (200) includes a base unit housing (210) to which the sensor unit (100) is coupled, and electronic components installed inside the base unit housing (210). The electronic component may include a circuit board (223), a base unit electrical contact portion (225) in contact with the sensor unit-electrical contact portion (146) of the sensor unit (100), a battery (228), etc. The circuit board (223) may be equipped with a processor chip for processing signals, a communication chip for wireless communication with the external terminal 5, etc.

The base unit housing (210) is provided with an insertion hole (211) through which the insertion portion (116) of the sensor (110) and the needle (450) can pass, and a mounting portion (212) in which the sensor unit housing (120) is coupled. The mounting portion (212) includes a base unit recess (213) and a contact surface (216) provided inside the base unit recess (213). The insertion hole (211) is shaped so that the boss (127) of the sensor unit (100) can be fitted and is disposed inside the base unit recess (213). The contact surface (216) may be flat so that the adhesive layer (155) of the sensor unit (100) can be stably adhered. The base unit recess (213) includes a first recess (214) connected to the insertion hole (211), and a second recess (215) that is located farther from the insertion hole (211) than the first recess (214) and is connected to the first recess (214). The second recess (215) is wider than the first recess (214). The first recess (214) can be made in a shape corresponding to the body portion (143) of the sensor unit housing (120), and the second recess (215) can be made in a shape corresponding to the cover portion (144) of the sensor unit housing (120). Therefore, the sensor unit housing (120) can be fitted into the base unit recess (213) and maintain a stable coupling state with the base unit housing (210). In addition, since the sensor unit housing (120) is stably fitted and coupled to the base unit housing (210), moisture or foreign substances cannot easily enter the gap between the sensor unit housing (120) and the base unit housing (210). A housing groove (217) is formed on the outer edge of the base unit housing (210). Some part of a locking hook (360) provided on the applicator (30) may be inserted into the housing groove (217).

The base unit-electrical contact portion (225) is arranged on the mounting portion (212) and contacts the sensor unit-electrical contact portion (146) of sensor unit (100) when sensor unit (100) is coupled to the base unit (200). The base unit-electrical contact portion (225) is electrically connected to the circuit board (223) and is installed in the base unit housing (210) so that a portion is exposed to the base unit recess (213). The base unit-electrical contact portion (225) may include a plurality of terminal portions (226) for transmitting electrical signals. The terminal portion (226) may electrically connect the sensor unit-electrical contact portion (146) and the circuit board (223) by contacting the terminal portion (147) of the sensor unit (100).

As shown, the base unit housing (210) may include a lower housing (219) and an upper housing (221) that covers the upper part of the lower housing (219), but its shape may be changed in various ways.

An adhesive portion (230) is provided on the surface of the base unit housing (210). The adhesive portion (230) is attached to the surface of the lower housing (219) to adhere the base unit housing (210) to the skin of a user. In the middle of the adhesive portion (230), an adhesive hole (231) through which the insertion portion (116) of the sensor (110) and the needle (450) can pass is formed to penetrate the adhesive portion (230) in the thickness direction.

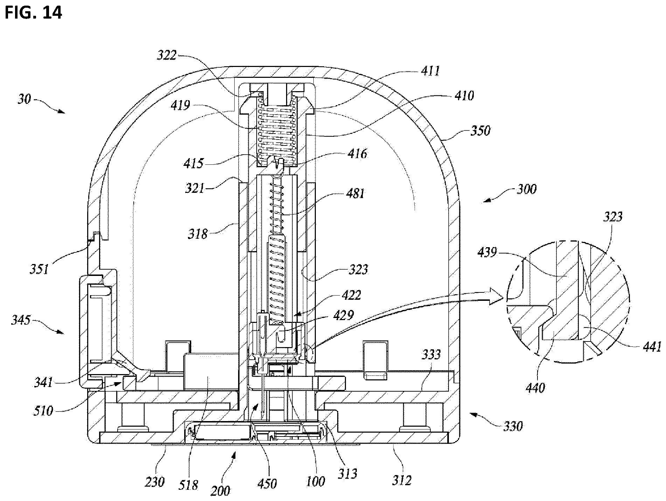

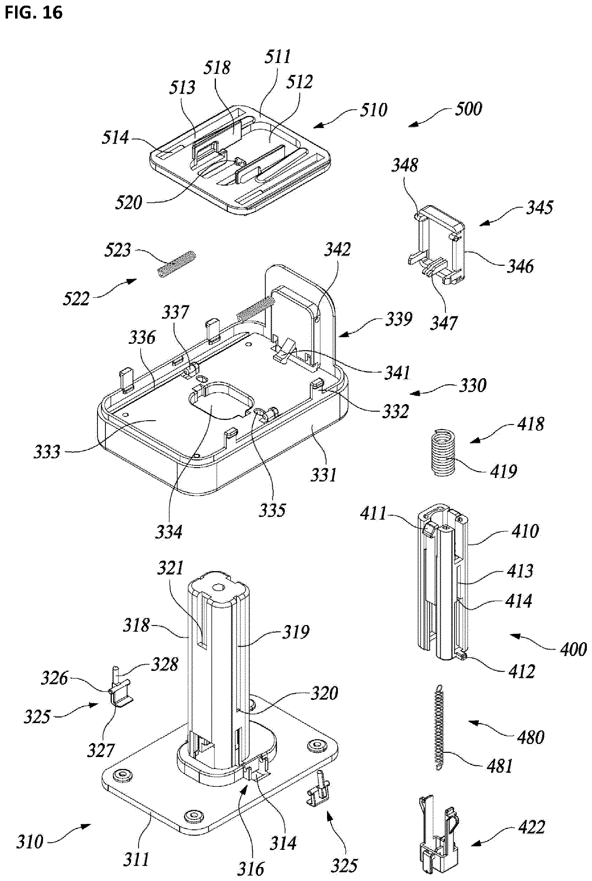

The adhesive portion (230) may be covered and protected with a protective sheet. The protective sheet covering the adhesive portion (230) may be removed during the process of attaching the base unit (200) to the skin of a user. The sensor unit (100) and the base unit (200) are installed in the applicator (30) in a separated state, and are coupled to each other in the process of operating the applicator (30) to insert the sensor (110) into the skin of a user, forming the body attachable unit (20). The sensor unit (100) may be mounted on the applicator (30) while the needle (450) coupled, and moved toward the base unit (200) with the needle (450) coupled thereto to be coupled to the base unit (200). After the sensor unit (100) is coupled to the base unit (200), the needle (450) is separated from the sensor unit (100), and only the body attachable unit (20) remains on the skin of a user. Referring to FIGs 14 to 16, the applicator (30) operates with the sensor unit (100) and the base unit (200) mounted, thereby coupling the sensor unit (100) and the base unit (200) and can attach the body attachable unit (20) in which the sensor unit (100) and the base unit (200) are coupled to the skin of a user. The sensor unit (100) and the base unit (200) are spaced apart from each other and are respectively mounted on the applicator (30). The applicator (30) is separated from the body attachable unit (20) after attaching the body attachable unit (20) to the skin of a user, and the body attachable unit (20) remains attached to the skin of a user by the adhesive portion (230).

The applicator (30) includes an applicator body (300) to which the base unit (200) is detachably coupled, an insertion unit (400) that moves the sensor unit (100) to insert the sensor (110) into the skin of a user, and removing unit (500) for removing the protective sheet (160) of the sensor unit (100). The insertion unit (400) may move the sensor unit (100) from a first position spaced apart from the base unit (200) by a preset distance to a second position coupled with the base unit (200). The sensor unit (100) and a top case (350) which is coupled to the middle frame (330) and covers the upper part of the middle frame (330).

The applicator body (300) includes a base frame (310) on which the insertion unit (400) is installed, a middle frame (330) disposed on the base frame (310) and on which the removing unit (500) is installed, and a top case (350) that is coupled to and covers the upper part of the middle frame (330).

The base frame (310) includes a frame base portion (311) having a bottom portion (312) that can be in contact with the skin of a user, and a column portion (318) which protrudes from the frame base portion (311) and accommodates the sensor unit (100) and the insertion unit (400). The bottom portion (312) of the frame base portion (311) is provided with a recess (313) into which the base unit (200) is mounted. The base unit (200) is detachably coupled to the recess (313) so that the adhesive portion (230) can face the skin of a user and placed in a second position spaced apart from the sensor unit (100). A through hole (314) is formed in the frame base portion (311) to penetrate the frame base portion (311) in the thickness direction. A pair of through holes (314) are provided on both sides of the column portion (318) with the column portion (318) interposed therebetween. A support portion (316) to which the locking hook (325) is coupled is provided at the top of the through hole (314).

Slits (319) are formed on both sides of the column portion (318) in a direction parallel to the moving direction of the sensor unit (100). A release portion (320) is provided in the middle of the slit (319). The release portion (320) is a portion of the carrier (422) that can be contacted while the carrier (422) of the insertion unit (400) moves toward the base unit (200). The carrier (422) may be disengaged from the plunger (410) by the action of the release portion (320). The specific operation of the release portion (320) will be described later. A detent portion (321) is provided on one side of the column portion (318) to limit the moving distance of the plunger (410). A retention unit (322) is provided inside the column unit (318). The retention unit (322) serves to support the plunger driver (418), which provides moving force to the plunger (410).

A locking hook (325) engaged with the base unit (200) mounted in the recess (313) is coupled to the support portion (316) of the base frame (310). The locking hook (325) engages the base unit (200) and fixes the base unit (200) so as not to be separated from the recess (313). The locking hook (325) may be disengaged from the base unit (200) after the body attachable unit (20) is attached to the skin of a user. The locking hook (325) is pivotally coupled to the support portion (316). The locking hook (325) includes a pivot portion (326) rotatably coupled to the support portion (316), a hook portion (327) connected to the pivot portion (326), and a rod (328) protruding from the pivot portion (326). The hook portion (327) may be inserted into the housing groove (217) of the base unit (200) and engaged with the base unit (200). The hook portion (327) protrudes from one side of the pivot portion (326), and the rod (328) protrudes from the other side of the pivot portion (326). The hook portion (327) is inserted into the through hole (314) of the base frame (310), and a portion of it is located in the recess (313) of the base frame (310). The locking hook (325) may be disengaged from the base unit (200) in conjunction with the moving member (510) of the removing unit (500). A more specific operation of the locking hook (325) will be described later.

The specific configuration of the base frame (310) is not limited to that shown and can be changed in various ways.

The middle frame (330) includes an outer frame (331) coupled to the base frame (310), a stage (333) disposed inside the outer frame (331), and a support fixture (339) protruding from the stage (333). The outer frame (331) is provided with a hook portion (332) that can be engaged with the top case (370). The stage (333) is disposed on the frame base portion (311). A middle frame opening (334) into which the column portion (318) is inserted is provided in the middle of the stage (333). On both sides of the middle frame opening (334), a through hole (335) is formed into which the rod (363) of the locking hook (360) is inserted. The rod (363) may pass through the through hole (335) and protrude onto the stage (333). The stage (333) is provided with a guide rail (336) and a fixing protrusion (337). The guide rail (336) serves to guide the moving member (510) to move linearly. The fixing protrusion (337) supports the moving member driver (522) that provides moving force to the moving member (510). The support fixture (339) is provided with an elastically deformable stopper (341) for restraining the movement of the moving member (510).

An operating member (345) capable of operating the insertion unit (400) and the removing unit (500) is installed on the support fixture (339). The operating member (345) includes a button portion (346), a pressing protrusion (347) protruding from the button portion (346) so as to contact the stopper (341), and a pivot portion (348) inserted into the coupling hole (342) of the support (339). The operating member (345) may rotate around the pivot portion (348). When a user presses the button portion (346), the operating member (345) rotates so that the pressing protrusion (347) presses the stopper (341). At this time, the stopper (341) may be elastically deformed and disengaged from the moving member (510). In addition to the configuration shown, the operating member (345) can be changed to various different configurations in which it is installed on the applicator body (300) to be operated by a user and can operate the insertion unit (400) and the removing unit (500).

The specific configuration of the middle frame (330) is not limited to that shown and can be changed in various ways. As another exemplary embodiment, the middle frame (330) may be formed integrally with the base frame (310).

The top case (350) is coupled with the middle frame (330) and covers the middle frame (330) and the upper portions of the base frame (310). On one side of the top case (350), a top case opening (351) is provided in a shape which allows the support fixture (339) of the middle frame (330) to be coupled. The top case (350) has a coupling hole (352) and can be coupled to the middle frame (330) by engaging the hook portion (332) of the middle frame (330) with the coupling hole (352).

The specific configuration of the top case (350) is not limited to that shown and can be changed in various ways. Additionally, the method of coupling the top case (350) and the middle frame (330) can also be changed in various ways.

The insertion unit (400) is installed on the applicator body (300) to associate with the moving member (510) of the removing unit (500) to move the sensor unit (100) from the first position to the second position, and the sensor (110) can be inserted into the skin of a user. The insertion unit (400) includes a plunger (410) installed movably on the middle frame (330) and a needle assembly (421) having a needle (450) that can be moved with the plunger (410) and inserted into the skin of a user.

The plunger (410) is installed inside the column portion (318) to be movable from the first position to the second position. A plunger hook (411) is provided on one side of the plunger (410) to limit the movement range of the plunger (410). The plunger hook (411) protrudes from the plunger (410) and can limit the movement of the plunger (410) by contacting the detent portion (321) of the column portion (318) when the plunger (410) moves to the second position. A plunger protrusion (412) is provided on the other side of the plunger (410). The plunger protrusion (412) protrudes from the plunger (410) to engage with the moving member (510). The plunger (410) is fixed in the first position when the plunger protrusion (412) is engaged with the moving member (510), and can be moved to the second position when the plunger protrusion (412) is disengaged from the moving member (510).

A pair of slits (413) are formed on both sides of the plunger (410) parallel to the moving direction of the sensor unit (100). A detent portion (414) with which the latch portion (437) of the carrier (422) can be engaged is provided in the middle portion of the slit (413). In a state where the latch portion (437) is engaged with the detent portion (414), the carrier (422) moves together with the plunger (410) and cannot move relative to the plunger (410).

A retention portion (415) and a fixing portion (416) are provided inside the plunger (410). The retention portion (415) supports the plunger driver (418). A needle release driver (480) that provides a moving force to the carrier (422) is coupled to the fixing portion (416).

The plunger (410) moves by a moving force from the plunger driver (418). The plunger driver (418) includes an elastic member (419) that applies elastic force to the plunger (410) in a direction of moving from the first position to the second position. The elastic member (419) has one end in contact with the retention portion (322) of the column portion (318) and the other end in contact with the retention portion (415) of the plunger (410). The plunger (410) may be fixed in the first position by engaging the moving member (510) in a state in which the elastic member (419) is elastically deformed. The plunger driver (418) may be changed to various other configurations that can provide a moving force to the plunger (410) in addition to the configuration including the elastic member (419) in the form of a coil spring.

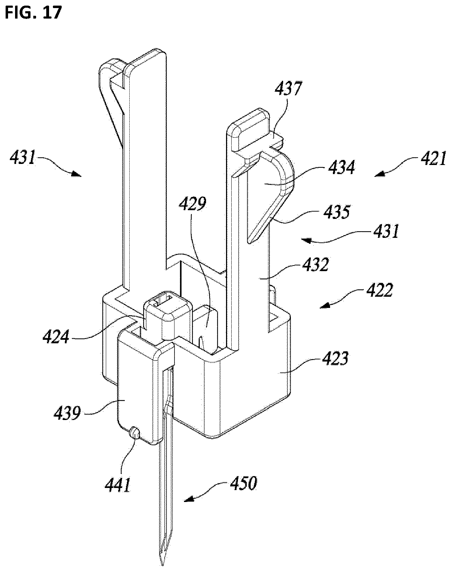

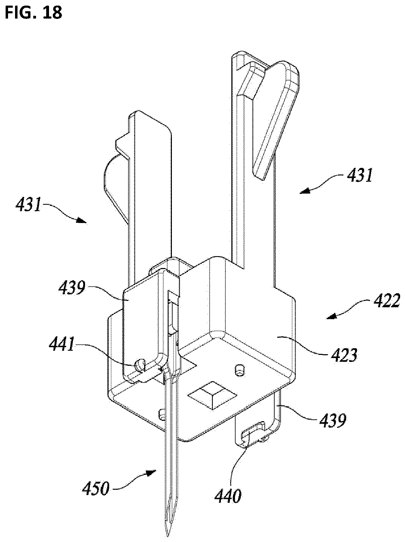

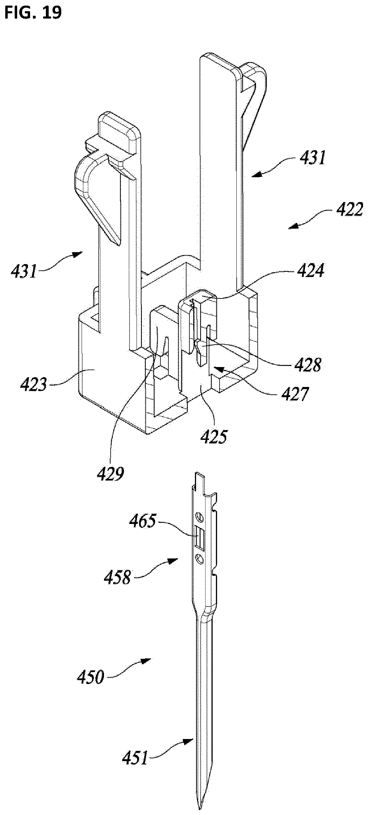

Referring to FIGS. 14 to 19, the needle assembly (421) includes the carrier (422) to which the sensor unit (100) is detachably coupled, and a needle (450) coupled to the carrier (422) penetrating the sensor unit (100). In a state in which the needle (450) is coupled to the sensor unit (100), the needle (450) advance from the first position to the second position together with the sensor unit (100) to be inserted into the skin of a user, and then may retreat with the carrier (422) in the second position and be separated from the skin of a user and the sensor unit (100).

The carrier (422) is coupled to the plunger (410) to enable relative movement. A portion of the carrier (422) engages the plunger (410) so as to move with the plunger (410) from the first position to the second position. Then, the carrier (422) is disengaged from the plunger (410) in the second position and may retreat in a direction away from the sensor unit (100).

The carrier (422) includes a carrier body (423) to which the needle (450) is coupled, an elastically deformable carrier wing (431) extending from the carrier body (423), and a locking arm (439) connected to the side of the carrier body (423).

The carrier body (423) includes a boss (424) with an insertion groove (425) formed therein. A portion of the needle (450) may be inserted into the insertion groove (425). A clamp portion (427) for fixing the needle (450) is provided in the insertion groove (425). The clamp portion (427) includes a hook (428) that can engage with the needle (450). A fixing portion (429) to which the needle release driver (480) is coupled is provided on one side of the carrier body (423). The carrier (422) can be moved relative to the plunger (410) by the needle release driver (480).

The needle release driver (480) provides a moving force to the carrier (422). The needle release driver (480) includes an elastic member (481) whose one end is coupled to the fixing portion (416) of the plunger (410) and the other end is coupled to the fixing portion (429) of the carrier (422). The elastic member (481) is in the form of a tension spring and applies an elastic force to pull the carrier (422) toward the fixing portion (416) of the plunger (410). That is, the elastic member (481) can apply an elastic force to the carrier (422) in a direction away from the sensor unit (100) to move the needle (450) coupled to the carrier (422) to be separated from the sensor unit (100). The needle release driver (480) may be changed to various other configurations that can provide a moving force to the carrier (422) in addition to the configuration including the elastic member (481) in the form of a coil spring.

The carrier wing (431) is connected to the carrier body (423) in a shape that extends from the carrier body (423) in the moving direction of the carrier (422). The carrier wing (431) includes a wing body (432) elastically deformably connected to the carrier body (423), and a trigger (434) and a latch portion (437) protruding from the wing body 432.

The trigger (434) is provided on one side of the wing body (432) to protrude outward from the wing body (432). The trigger (434) is inserted into the slit (413) of the plunger (410) and the slit (319) of the column portion (318) and can move along these slits (413) and (319). The trigger (434) is inserted into the slits (413 and 319) and guided by the slits (413 and 319), so that the carrier (422) can move linearly more stably. The trigger (434) passes through the slit (413) of the plunger (410), and some part is inserted into the slit (319) of the column portion (318). While the carrier (422) moves with the plunger (410) from the first position to the second position, the trigger (434) may contact the release portion (320) disposed in the middle of the slit (319) of the column portion (318).

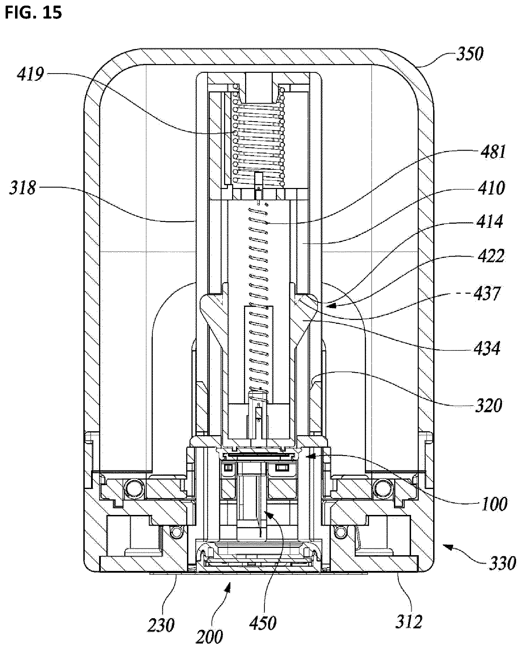

As shown in FIG. 15, the release portion (320) is disposed in the middle of the slit (319) of the column portion (318) to press the trigger (434). While the carrier (422) is moving from the first position to the second position, the trigger (434) may come into contact with the release portion (320) and the carrier wing (431) may be elastically deformed. The trigger (434) is provided with a trigger inclined portion (435) that contacts the release portion (320). While the carrier (422) moves from the first position to the second position, the trigger inclined portion (435) comes into contact with the release portion (320), so that the carrier wing (431) can be elastically deformed more smoothly, and the impact that occurs when the trigger (434) comes into contact with the release portion (320) is reduced, and the pressing force of the release portion (320) can be more smoothly transmitted to the trigger (434).

The latch portion (437) protrudes outward from the wing body (432). The direction in which the latch portion (437) protrudes from the wing body (432) is the same as the direction in which the trigger (434) protrudes from the wing body (432). The protrusion height at which the latch portion (437) protrudes from the wing body (432) is lower than the protrusion height at which the trigger (434) protrudes from the wing body (432). In a state in which the latch portion (437) is engaged with the detent portion (414), the carrier (422) can maintain the elastic member (481) in an elastically deformed state, and cannot move in the direction in which the elastic force of the needle release driver (480) acts.

The locking arm (439) is provided on the side of the carrier body (423) to be elastically deformable. A pair of locking arms (439) are arranged to face each other on both sides of the carrier body (423) and detachably fix the sensor unit housing (120) of the sensor unit (100). The locking arm (439) is provided with a locking protrusion (440) engaged with the sensor unit housing (120) and a protrusion (441) in contact with an inner wall (323) of the column portion (318). As shown in FIG. 14, when the carrier (422) and the sensor unit (100) are located in the first position, as the protrusion (441) contacts the inner wall (323) of the column portion (318), the locking arm (439) is biased in a direction in which it engages with the sensor unit housing (120). Accordingly, the sensor unit (100) can remain stably coupled to the carrier (422) in the first position.

Meanwhile, when the sensor unit (100) and the carrier (422) move toward the base unit (200) and the sensor unit (100) is coupled with the base unit (200), the protrusion (441) is located in a space connected to the recess (313) in which the base unit (200) is accommodated and deviates from the inner wall (323) of the column portion (318). Therefore, when the sensor unit (100) is coupled to the base unit (200), the fixing force of the locking arm (439) is weakened. And the sensor unit (100) is fixed to the base unit (200) by the adhesive layer (155), so that when the carrier (422) is pulled in a direction away from the body attachable unit (20) by the needle release driver (480), the locking arm (439) can be smoothly disengaged from the sensor unit (100) and separated from the sensor unit (100).

In addition to the configuration shown, the carrier (422) can be changed to various other configurations in which it is coupled to the needle (450) and installed to be relatively movable on the plunger (410). For example, the drawing shows a pair of carrier wings (431) being symmetrically provided on both sides of the carrier body (423), but the number and shape of the carrier wings (431) can be changed in various ways. Additionally, the coupling method of the carrier (422) and the needle (450) and the coupling method of the carrier (422) and the sensor unit (100) may also be changed in various ways.

The needle (450) is fixed to the carrier (422) and can move from the first position to the second position while coupled to the sensor unit (100). The needle (450) has a sharp tip so as to pierce the skin of a user and be smoothly inserted into the skin of a user. When the sensor unit (100) moves to the second position, the needle (450) penetrates the skin of a user before the sensor (110) and allows the sensor (110) to be stably inserted into the skin. The needle (450) is separated from the skin of a user after the sensor (110) is inserted into the skin of a user.

The needle (450) includes a needle body (451) inserted into the skin of a user of a user and a needle head (458) coupled to the carrier (422). The needle head (458) is provided with a needle hole (465) that engages the clamp portion (427) of the carrier (422). The needle (450) may be fixed to the carrier (422) in such a way that the needle head (458) is inserted into the insertion groove (425) of the carrier (422) and the hook (428) is inserted into the needle hole (465). The needle (450) may be formed as one piece by press-processing the needle body (451) and the needle head (458) from the same base material. The needle (450) has a simple structure, is easy to manufacture, and has a low manufacturing cost. And the needle (450) can be simply assembled to the carrier (422) by simply inserting the needle head (458) into the insertion groove (425) of the carrier (422). When the needle head (458) is inserted into the insertion groove (425) to a certain depth, the hook (428) of the carrier (422) is inserted into the needle hole (465) of the needle head (458), so that the needle (450) can be firmly fixed the carrier (422).

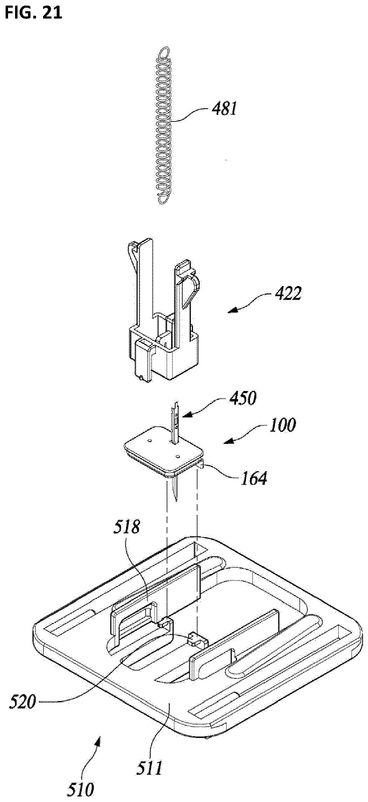

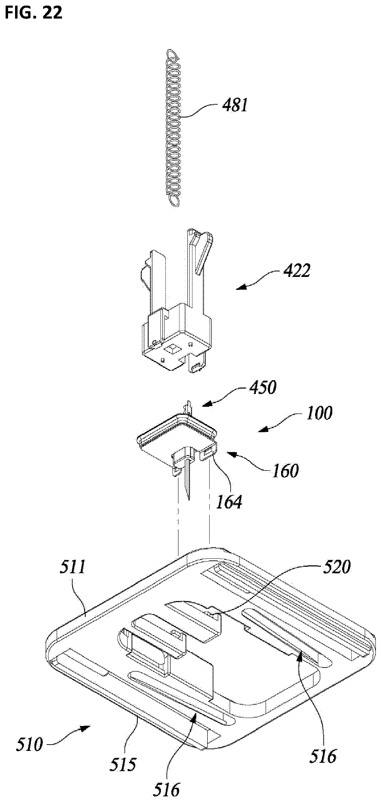

Referring to FIGS. 14 to 16 and FIGS. 20 to 30, before the sensor unit (100) reaches the second position and is coupled to the base unit (200), the removing unit (500) is installed on the applicator body (300) to separate the protective sheet (160) of the sensor unit (100) from the adhesive layer (155). The removing unit (500) includes a moving member (510) installed movably on the middle frame (330) to be coupled to the protective sheet (160) and a moving member driver (522) that moves the moving member (510) by providing a moving force to the moving member (510).

The moving member (510) is arranged to move linearly on the stage (333) of the middle frame (330). The moving member (510) is installed to move in a direction that is approximately perpendicular to the direction in which the sensor unit (100) moves from the first position to the second position. The moving member (510) may pull the protective sheet (160) to be separated from the adhesive layer (155) by moving while coupled with the protective sheet (160). Specifically, in a state in which the protective sheet (160) covers the adhesive layer (155), the moving member (510) can move from a third position where the protective sheet is coupled to a fourth position where the protective sheet (160) is removed from the adhesive layer. Additionally, the moving member (510) may act as a stopper that fixes the plunger (410) in the first position. That is, the moving member (510) fixes the plunger (410) at the first position by coming into contact with the plunger (410) located in the first position at the third position, and when the moving member (510) moves to the fourth position, it deviates from the plunger (410) so that the plunger (410) can move to the second position.

The moving member (510) includes a moving member body (511), a supporting portion (518) for supporting the plunger (410), and a grip portion (520) coupled with the protective sheet (160).

A moving member opening (512) and a moving member groove (513) are formed in the moving member body (511) to penetrate the moving member body (511) in the thickness direction. The moving member opening (512) is formed in the middle of the moving member body (511), and a pair of moving member grooves (513) are disposed on both sides of the moving member body (511). A fixing protrusion (514) connected to the moving member driver (522) is provided in the moving member groove (513). A rail portion (515) is provided on one surface of the moving member body (511) facing the stage (333) of the middle frame (330). The rail portion (515) may engage with the guide rail (336) of the middle frame (330) and slide along the guide rail (336). In the drawing, the guide rail (336) is shown in a groove shape and the rail portion (515) is inserted into the guide rail (336), but the guide rail (336) and the rail portion (515) can be changed into various other configuration that can guide the moving member body (511) to stably move linearly on the stage (333).

A pair of supporting portions (518) are disposed at both edges of the moving member opening (512) to face each other. The supporting portion (518) may protrude from the moving member body (511) and support the plunger protrusion (412) of the plunger (410). The length of the supporting portion (518) is shorter than the distance that the moving member body (511) moves from the third position to the fourth position. When the moving member (510) is located in the third position, the supporting portion (518) may support the plunger protrusion (412) of the plunger (410) and fix the plunger (410) in the first position. And when the moving member (510) moves to the fourth position, the supporting portion (518) deviates from the plunger protrusion (412) so that the plunger (410) can move to the second position. The supporting portion (518) may be changed into various other shapes that can support the plunger protrusion (412) or a portion of the plunger (410) in addition to the shape that protrudes from the moving member body (511).

The grip portion (520) is connected to the moving member body (511) so as to be located in the moving member opening (512). A portion of the grip portion (520) is inserted into the protective sheet groove (165) of the protective sheet (160), thereby being coupled to the wing portion (164) of the protective sheet (160). The method of coupling the moving member (510) and the protective sheet (160) can be changed to various methods other than using the grip portion (520). As another exemplary embodiment, the moving member (510) may be coupled to the protective sheet (160) through an adhesive method, or may be coupled to the protective sheet (160) by engaging an edge of the protective sheet (160).

The moving member driver (522) provides a moving force to the moving member (510) so that the moving member (510) can be moved from the third position to the fourth position. The moving member driver (522) includes an elastic member (523) that applies elastic force to the moving member (510) in a direction that is biased to the fourth position. The elastic member (523) is inserted into the moving member groove (513) of the moving member (510), so that one end is connected to the fixing protrusion (337) of the middle frame 330 and the other end is connected to the fixing protrusion (514) of the moving member (510). The moving member driver (522) may be changed into various other forms that can provide a moving force to the moving member (510) in addition to configuration including the elastic member (523) in the form of a coil spring.

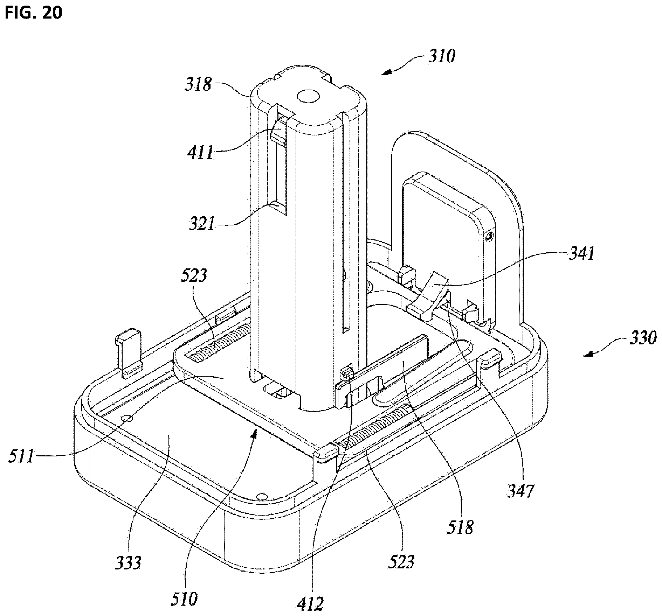

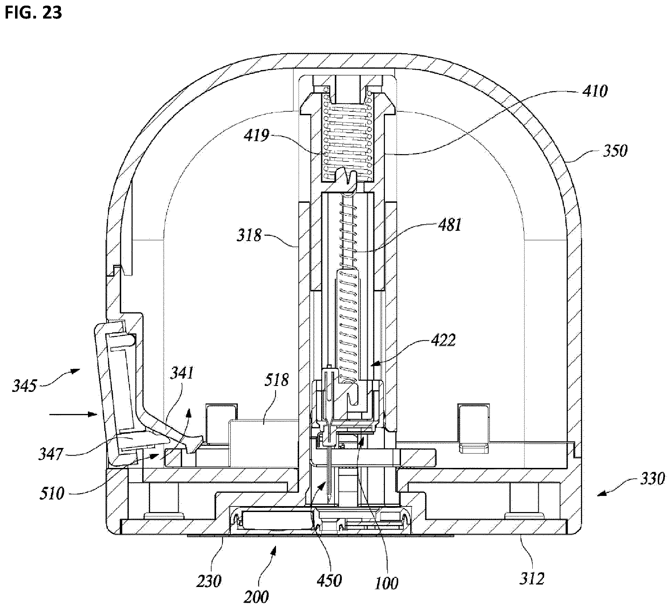

As shown in FIG. 20, the moving member (510) may engage the stopper (341) provided in the middle frame (330) at the third position and be fixed in a state in which the elastic member (523) is elastically deformed. A portion of the stopper (341) is inserted into the moving member opening (512) of the moving member (510) and engages with the moving member (510). The stopper (341) may be released by the operating member (345) to remove the binding force on the moving member (510). That is, as shown in FIG. 23, when a user presses the operating member (345), the operating member (345) can bias the stopper (341) to be disengaged from the moving member (510).

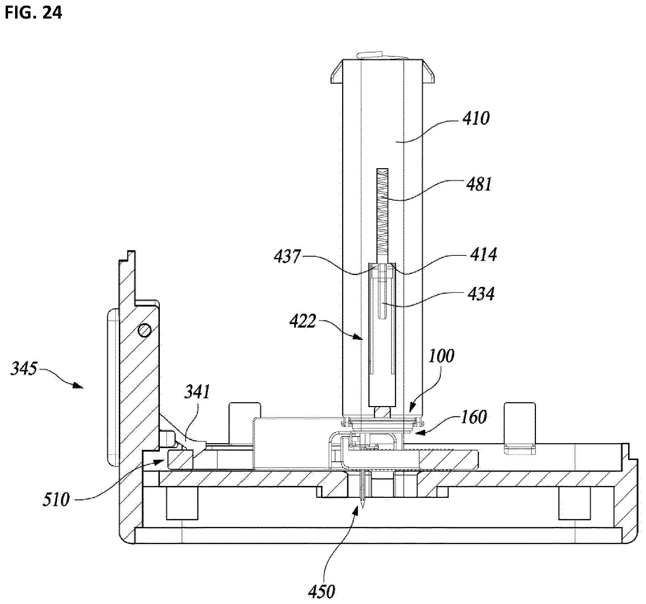

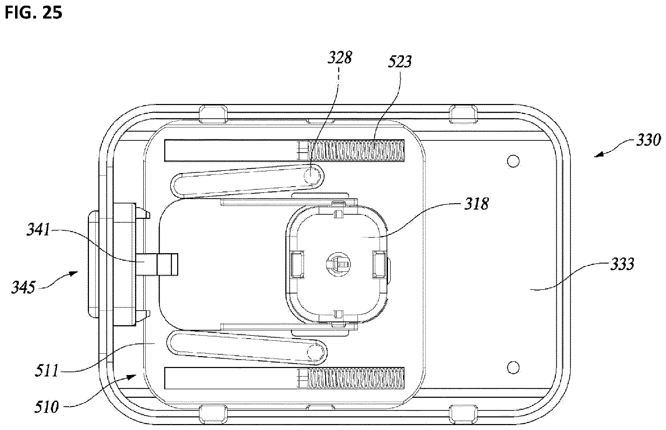

FIGS. 24 and 25 show a state in which the moving member (510) is engaged with the stopper (341) and fixed in the third position. At this time, the moving member (510) supports the plunger (410) and fixes the plunger (410) in the first position, and the protective sheet (160) coupled with the moving member (510) maintains the adhesive layer (155) covered.

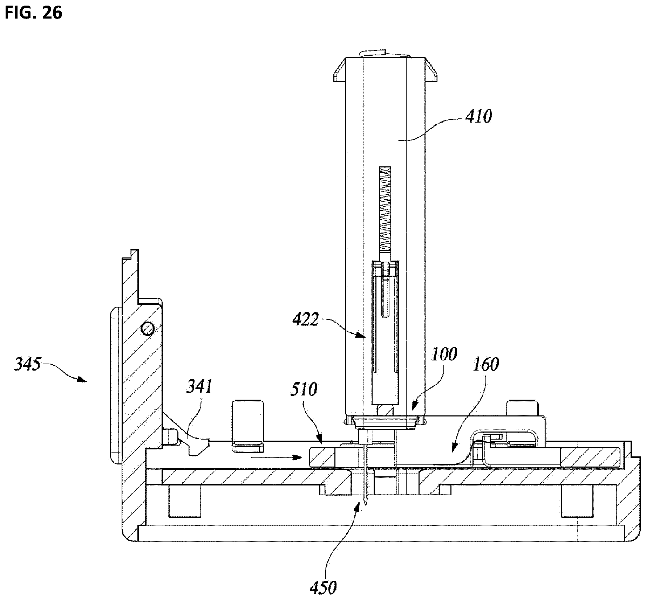

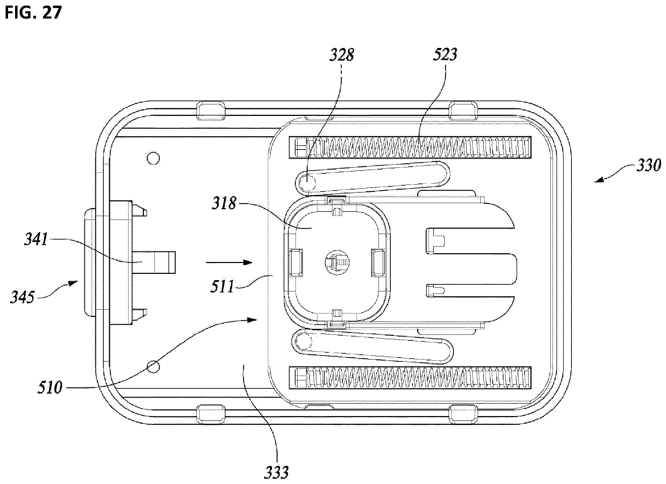

FIGS. 26 and 27 show the moving member (510) being disengaged from the stopper (341). At this time, the moving member (510) moves to the fourth position dragging the protective sheet (160). Accordingly, the protective sheet (160) is separated from the adhesive layer (155), and the plunger (410) can be moved out of the moving member (510) and moved to the second position by the plunger driver (418).

When the length of the support portion (518) supporting the plunger protrusion (412) of the plunger (410) is sufficiently long, the moving member (510) may be released from the plunger (410) after completely separating the protective sheet (160) from the adhesive layer (155). In this case, the separation operation of the protective sheet (160) is completed while the sensor unit (100) is located in the first position, and then the sensor unit (100) can be moved to the second position. Depending on the length of the supporting portion (518), it is possible that the plunger (410) starts to move toward the second position during the separation operation of the protective sheet (160).

In this way, the moving member (510) has the function of separating the protective sheet (160) and operating the insertion unit (400) in conjunction with the operating member (345). In addition to these functions, the moving member (510) can move the locking hook (325) that secures the base unit (200). For this purpose, a guide portion (516) engaged with the locking hook (325) is provided on one side of the moving member body (511) facing the stage (333) of the middle frame (330).

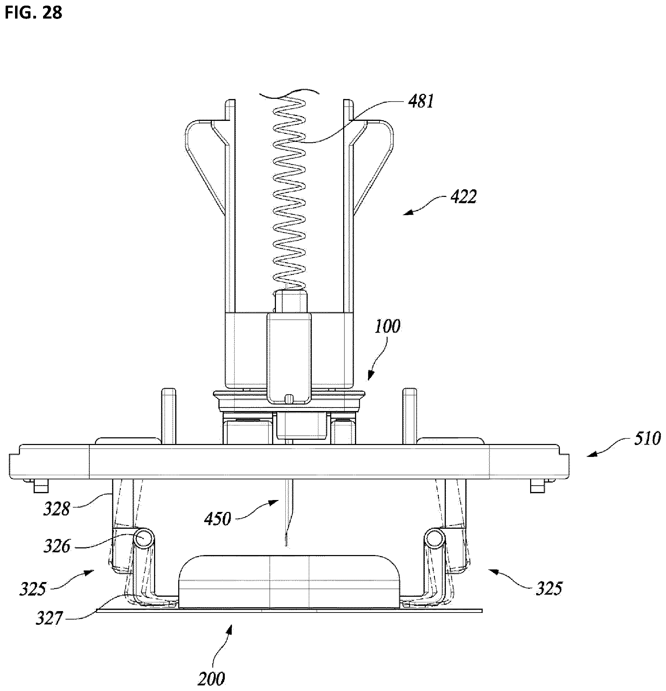

The guide portion (516) is formed in a groove shape that is disposed to be inclined with respect to the moving direction of the moving member (510) so that the end of a rod (328) of the locking hook (325) can be engaged. Since the end of the rod (328) is engaged with the guide portion (516), the locking hook (325) can rotate in conjunction with the moving member (510). As shown in FIG. 28, the locking hook (325) may rotate in the direction in which the hook portion (327) engages with the base unit (200) or rotate in a direction in which the hook portion (327) moves away from the base unit (200) depending on the position of the moving member (510).

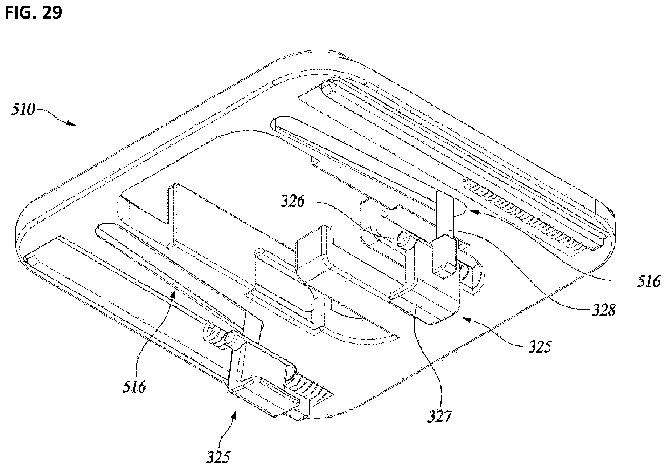

As shown in FIGS. 25 and 29, when the moving member (510) is located in the third position, the guide portion (516) biases the locking hook (325) in a direction in which the hook portion (327) engages with the base unit (200).

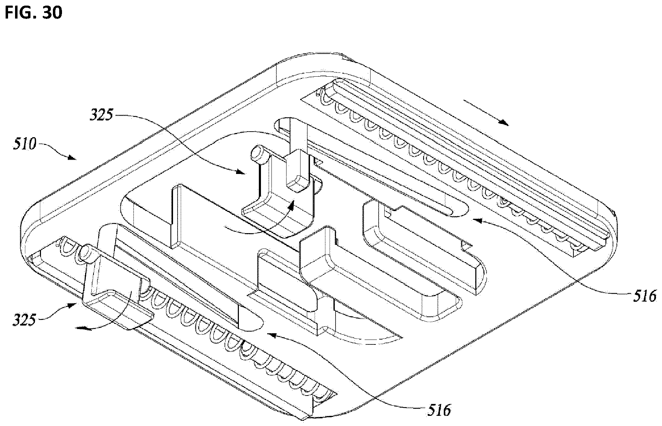

On the other hand, as shown in FIGS. 27 and 30, when the moving member (510) moves to the fourth position, the guide portion (516) rotates the locking hook (325) in a direction in which the hook unit (327) is disengaged from the base unit (200). That is, when the moving member (510) moves from the third position to the fourth position, the rod (328) of the locking hook (325) is pulled by the guide portion (516) in a direction approaching the moving member opening (512) of the moving member (510). At this time, by rotating around the pivot portion (326), the locking hook (325) is biased so that the hook portion (327) is disengaged from the base unit (200).

Due to the action of the movable member (510), when the movable member (510) is located in the third position, the locking hook (325) maintains a state of being stably engaged with the base unit (200) and may fix the base unit (200) so as not to be separated from the recess (313) of the applicator body (300) by maintaining a state in which the locking hook (325) is stably engaged with the base unit (200). And when the moving member (510) moves to the fourth position, the locking hook (325) is disengaged from the base unit (200) so that the base unit (200) can be separated from the applicator body (300). Therefore, after the sensor unit (100) is coupled with the base unit (200) and the body attachable unit (20) is assembled, the body attachable unit (20) can be smoothly separated from the applicator (30) while being attached to the skin of a user by the adhesive portion (230).

In the drawing, the guide portion (516) is shown to be in the form of a groove, but the guide portion is changed to various other shapes that engage the locking hook (325) and can bias the locking hook (325) according to the movement of the moving member (510). Depending on the shape of the guide portion, etc., the locking hook may also be changed into various other configurations that can be detachably engaged with the base unit (200).

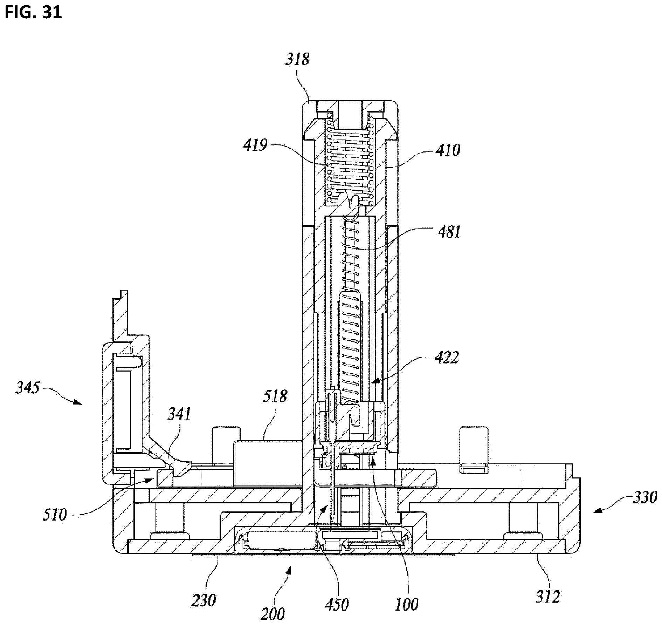

Hereinafter, with reference to FIGS. 31 to 36, using the applicator (30) according to an embodiment of the present disclosure, a specific process for attaching the body attachable unit (20) to the skin of a user will be described.

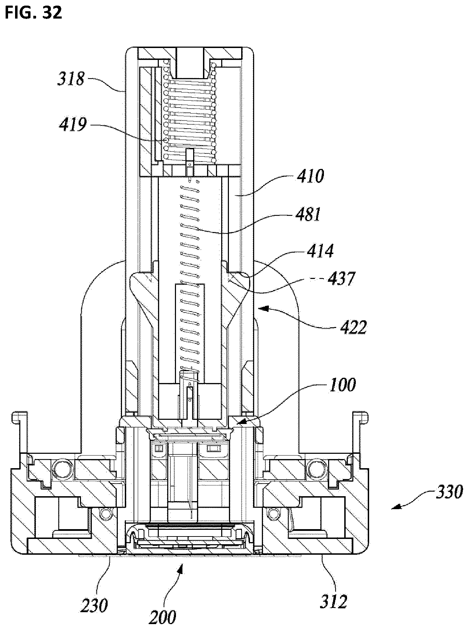

Referring to FIGS. 31 and 32, first, the bottom portion (312) of the applicator (30) is placed on the skin of a user so that the base unit (200) is attached to the skin of a user by the adhesive portion (230). Before the operating member (345) is manipulated, the sensor unit (100) coupled to the carrier (422) is positioned in the first position in a state in which the protective sheet (160) covers the adhesive layer (155). And the moving member (510) is located in the third position engaging with the stopper (341).

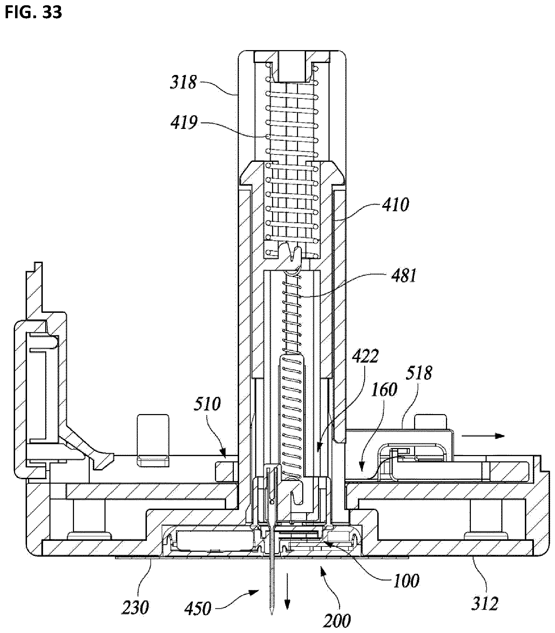

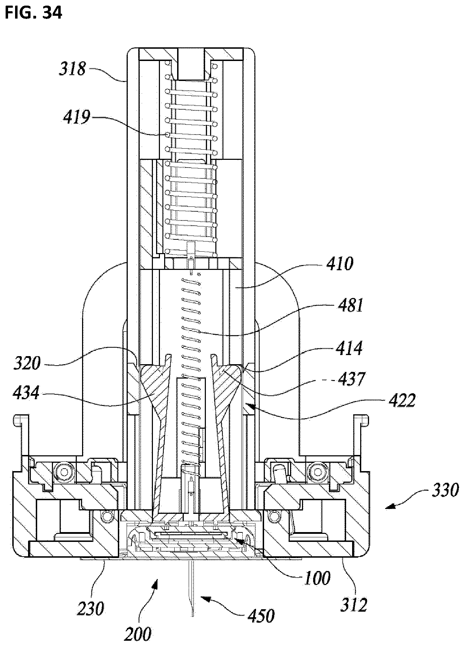

Thereafter, when the operating member (345) is pressed, the moving member (510) is disengaged from the stopper (341) and the moving member (510) is moved to the fourth position by the moving member driver (522). As shown in FIGS. 33 and 34, as the moving member (510) moves to the fourth position, the protective sheet (160) is pulled by the moving member (510) and separated from the adhesive layer (155). Then, the plunger (410) moves away from the moving member (510) and moves to the second position by the plunger driver (418). At this time, the insertion portion (116) of the sensor (110) and needle (450) are inserted into the skin of a user, and the sensor unit (100) from which the protective sheet (160) is separated is attached to the base unit (200) by the adhesive layer (155), so that the body attachable unit (20) is assembled.

As previously described, the adhesive layer (155) couples the sensor unit (100) and the base unit (200), and the adhesive layer (155) seals between the sensor unit-electrical contact portion (146) of the sensor unit (100) and the base unit-electrical contact portion (225) of the base unit (200). The adhesive layer (155) seals the electrical connection portion (250) between the sensor unit (100) and the base unit (200). In the process in which the body attachable unit (20) is attached to the skin of a user and measures biosignals, as the adhesive layer (155) seals the electrical connection portion (250) between the sensor unit (100) and the base unit (200), moisture, foreign substances, body fluids, etc. do not flow into the electrical connection portion (250) between the sensor unit (100) and the base unit (200). Therefore, the problem of malfunctioning of the body attachable unit (20) can be reduced, and stable operation of the body attachable unit (20) is possible.

When the plunger (410) moves and the insertion portion (116) and the needle (450) are inserted into the skin of a user, the trigger (434) of the carrier (422) comes into contact with the release portion (320) of the column portion (318). At this time, the carrier wing (431) of the carrier (422) is elastically deformed, so that the latch portion (437) of the carrier (422) is disengaged from the detent portion (414) of the plunger (410).

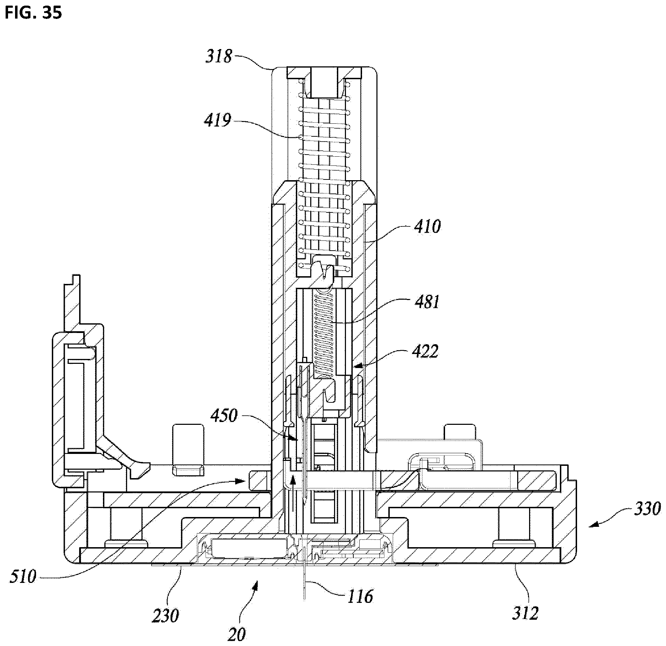

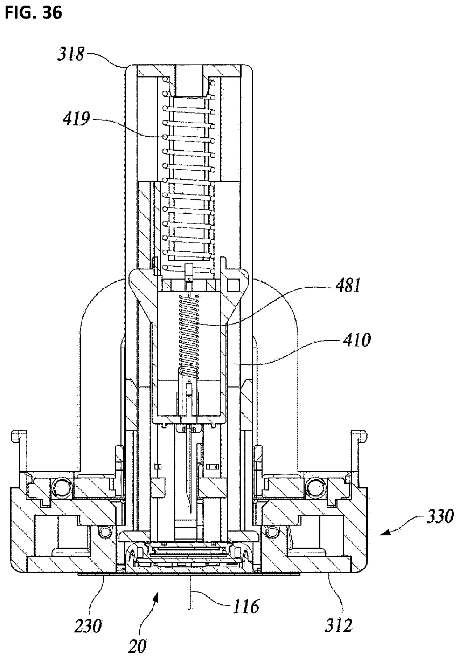

When the carrier (422) is disengaged from the plunger (410), the carrier (422) moves away from the skin of a user by the elastic member (481) of the needle release driver (480) as shown in FIGS. 35 and 36, At this time, the needle (450) comes out of the skin of a user and the body attachable unit (20) remains attached to the skin of a user by the adhesive portion (230).

As described above, in the process of the needle (450) coming out of the skin of a user after the insertion portion (116) of the sensor (110) is inserted into the skin, the retention protrusion (136) of the sensor unit housing (120) comes into contact with the middle portion (113) of the sensor (110) and restricts the movement of the insertion portion (116). Accordingly, the insertion portion (116) cannot retreat in the same direction as the needle (450) and can remain stably inserted into the skin.

In a state in which the sensor unit (100) is coupled with the base unit (200) and the body attachable unit (20) is assembled, the locking hook (325) engaged with the base unit (200) rotates in conjunction with the moving member (510). That is, the locking hook (325) is biased to be disengaged from the base unit (200) by the moving member (510). Accordingly, the body attachable unit (20) can be separated from the applicator (30) while being attached to the skin of a user.

The body attachable unit (20) attached to the skin of a user and separated from the applicator (30) can measure biometric information and transmit the measurement information to an external terminal (5) and the like.

As described above, the applicator assembly (10) according to an embodiment of the present disclosure is mounted on the applicator (30) with the adhesive layer (155) of the sensor unit (100) covered with the protective sheet (160), so that the adhesiveness of the adhesive layer (155) does not deteriorate even if it is not used for a long time. Then, as a user manipulates the operating member (345), the removing unit (500) operates to automatically remove the protective sheet (160) from the sensor unit (100), and the sensor unit (100) is firmly coupled to the base unit (200) by an adhesive layer (155). Therefore, it is convenient to use, and the body attachable unit (20) can be more stably attached to the skin of a user.

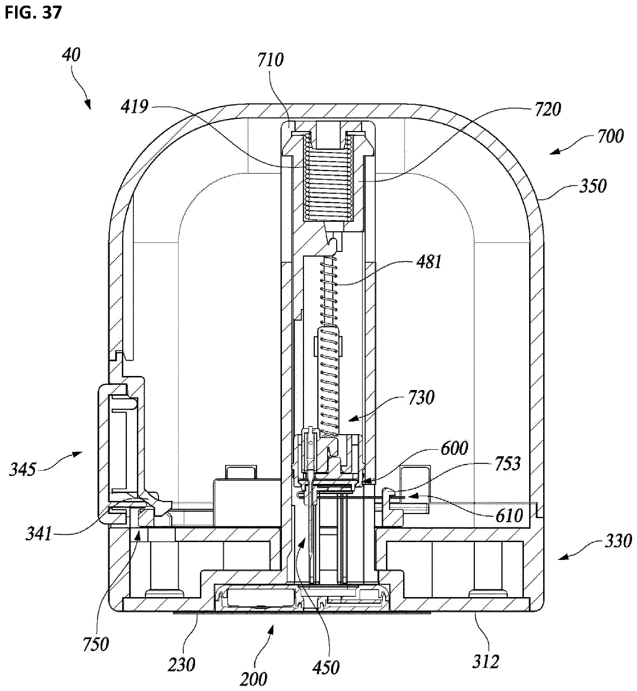

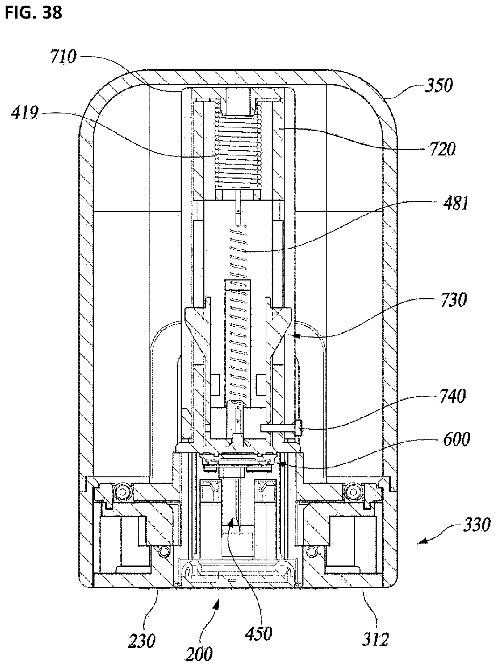

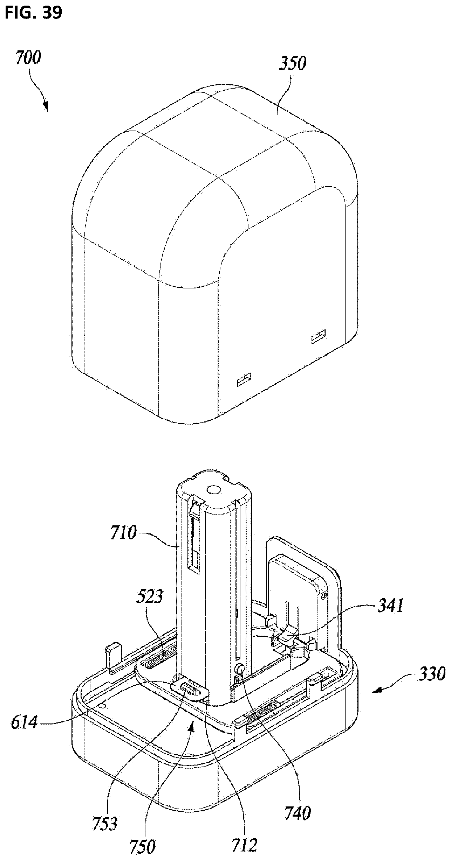

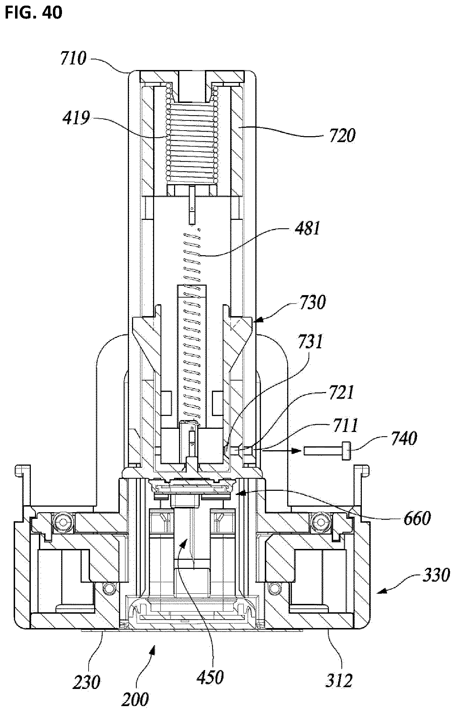

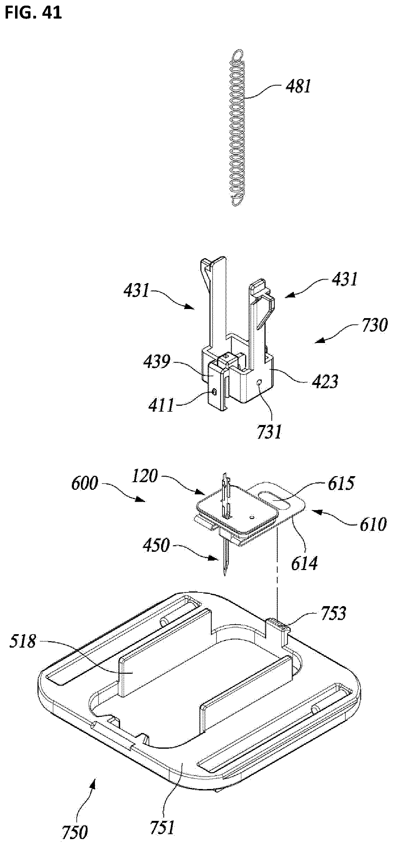

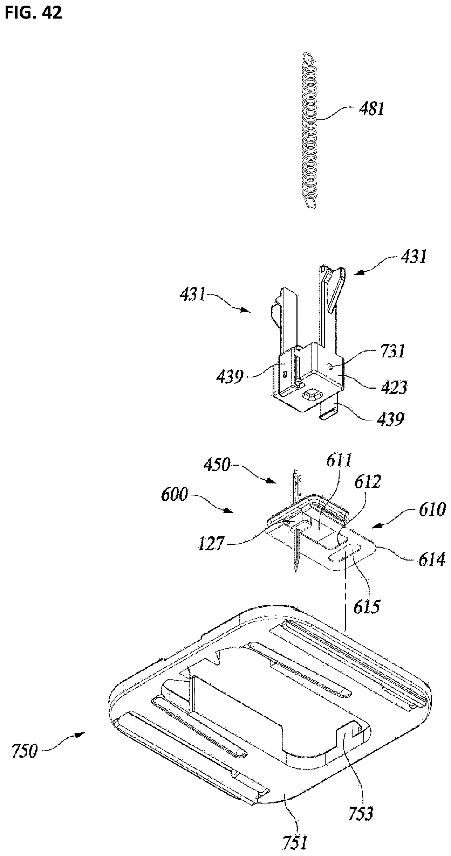

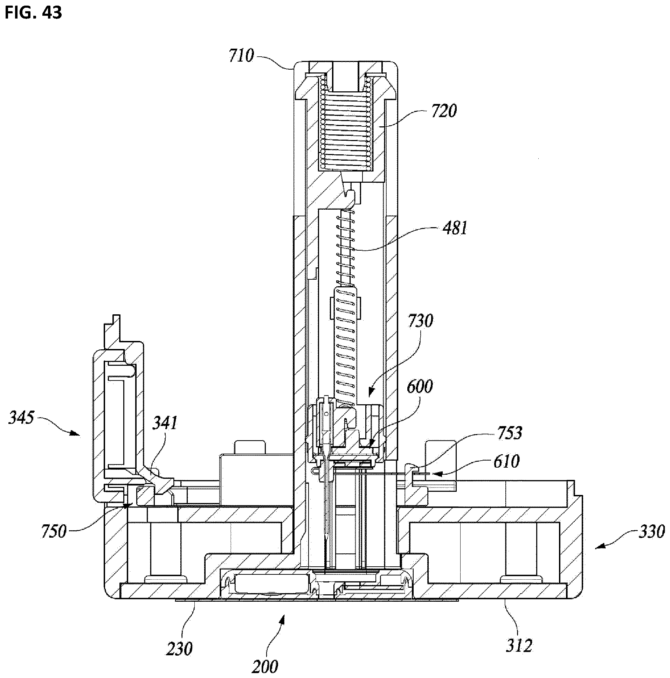

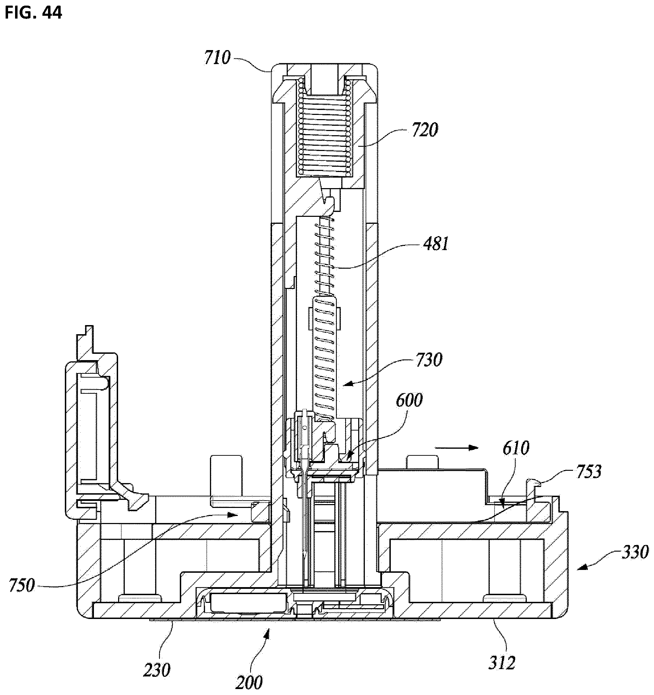

Meanwhile, FIGS. 37 to 44 show the applicator assembly according to another embodiment of the present disclosure.

An applicator assembly (40) according to another embodiment of the present disclosure includes a sensor unit (600) for measuring the biometric information of a user, the base unit (200) electrically connected to the sensor unit (600), and an applicator (700) for inserting the sensor of the sensor unit (600) into the skin of a user. In the applicator assembly (40), a partial configuration of the sensor unit (600) and a partial configuration of the applicator (700) are modified and most of the configurations are the same as those described above.

Like the sensor unit (100) described above, the sensor unit (600) includes the sensor inserted into the skin of a user, the sensor unit housing to which the sensor is coupled, the sensor unit-electrical contact portion that is coupled to the sensor unit housing so as to be electrically connected to the sensor, the sensor adhesive portion and the adhesive pad to secure the sensor to the sensor unit housing, the adhesive layer provided on the sensor unit housing (120) so as to be attached to the base unit (200) and a protective sheet (610) covering an adhesive layer, and the remaining configuration is the same as described above, except for the protective sheet (610).

The protective sheet (610) includes a protection portion (611) adhered to an adhesive layer, and a wing portion (614) extending from an edge of the protection portion (611). The length of the wing portion (614) is longer than the length of the protection portion (611). The wing portion (614) may be bent at one end of the protection portion (611) to completely cross the protection portion (611) and extend outward from the other end of the protection portion (611). A protective sheet opening (612) is formed on one side of the protection part (611) to penetrate the protection part (611) in the thickness direction. The protective sheet (610) is attached to the adhesive layer so that the boss (127) of the sensor unit (100) is inserted into the protective sheet opening (612). A protective sheet groove (615) is formed in the wing portion (614) to penetrate the wing portion (614) in the thickness direction. The wing portion (614) may extend by a certain distance from the edge of the sensor unit housing (120) to be coupled to the moving member (750) of the applicator (700). The protective sheet (610) is separated from the adhesive layer before the sensor unit (600) moves and reaches the base unit (200).

The applicator (700) operates in a state in which the sensor unit (600) and the base unit (200) are mounted to couple the sensor unit (600) and the base unit (200) and insert the sensor of the sensor unit (600) to the skin of a user. Most of the configuration of the applicator (700) is the same as described above, but partial configurations of column portion (710), plunger (720), carrier (730), and moving member (750) are modified. The applicator (700) further includes a safety pin (740) in a comparison of the applicator (30) described above.

The safety pin (740) can be inserted into a column portion hole (711) formed in the column portion (710), a plunger hole (721) formed in the plunger (720), and a carrier hole (731) formed in the carrier (730). When the plunger (720) is located in the first position, the column portion hole (711), the plunger hole (721), and the carrier hole (731) are aligned. In this state, the safety pin (740) can be inserted into the carrier hole (731) by sequentially passing through the column hole (711) and the plunger hole (721). The safety pin (740) may prevent movement of the plunger (720) and movement of the carrier (730) in the first position. Therefore, the safety pin (740) can prevent malfunction of the applicator (700) due to the user's carelessness. After separating the safety pin (740) from the column portion (710), the user may operate the applicator (700) to insert the sensor into the skin.

The specific configuration and number of safety pins (740) may vary. As another exemplary embodiment, the safety pin passing through the column portion may be installed to engage the plunger without reaching the carrier. In this case, the safety pin can prevent malfunction of the applicator due to the user's carelessness by restraining the movement of the plunger in the first position.

The moving member (750) is modified in the shape of a moving member body (751) and the position of a grip portion (753) in comparison with the moving member (510) described above. The grip portion (753) protrudes from one surface of the moving member body (751) in a direction intersecting the moving direction of the moving member body (751). The grip portion (753) is disposed outside the column portion (710) in a state in which the moving member (750) is fixed in the third position by the stopper (341). A portion of the wing portion (614) of the protective sheet (610) may pass through the opening (712) of the column portion (710) and be coupled to the grip portion (753) located outside the column portion (710).

The shape or location of the grip portion coupled with the protective sheet, or the location of the coupling portion between the moving member and the protective sheet may be changed in various ways. As another exemplary embodiment, the grip portion may be located on the side, edge, or bottom of the moving member body.

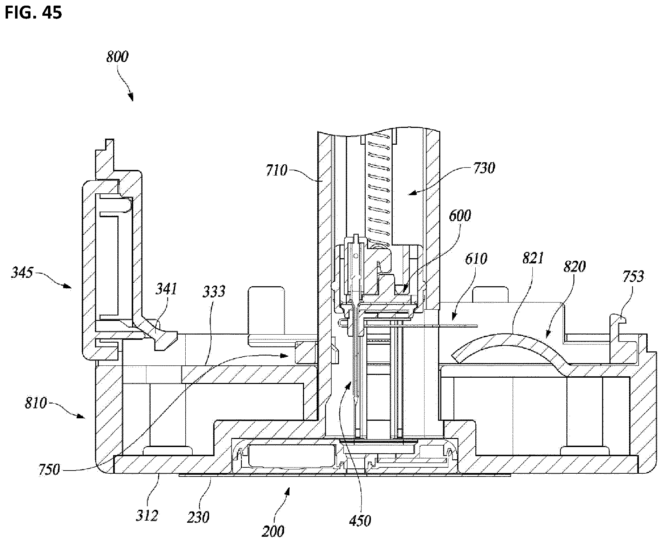

Meanwhile, FIG. 45 is a cross-sectional view showing a portion of an applicator according to another embodiment of the present disclosure.

An applicator (800) shown in FIG. 45 is a partially modified configuration of an applicator body (810).

The applicator body (810) includes a support (820) for supporting the protective sheet (610) separated from the adhesive layer of the sensor unit (600). In a case in which the protective sheet (610) separated by the moving member (750) is located in the moving path of the sensor unit (600), the carrier (730), or the plunger (720), while the applicator (800) is operating, the protective sheet (610) interferes with the sensor unit (600), the carrier (730), or the plunger (720) and may prevent the smooth operation of the applicator (800). In the applicator (800) according to this embodiment, after the separation operation of the protective sheet (610), the support (820) supports the protective sheet (610), so that it is possible to prevent a problem in which the protective sheet (610) interferes with components which are moving during the operation of the applicator (800), such as the sensor unit (600), the carrier (730), and the plunger (720).

As shown in FIG. 45, the support (820) has a curved arch portion (821) that can contact the protective sheet (610), and can be installed on the applicator body (810) to be biased upward. The support (820) may be elastically deformed by the moving member (750) and then return to its original state so as to be biased upward when it leaves the moving member (750).

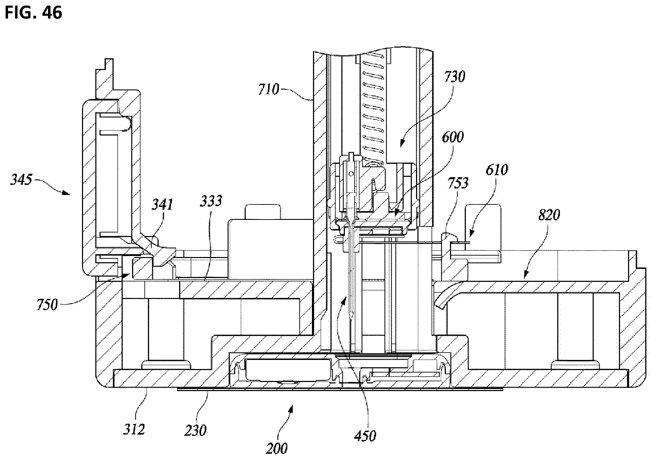

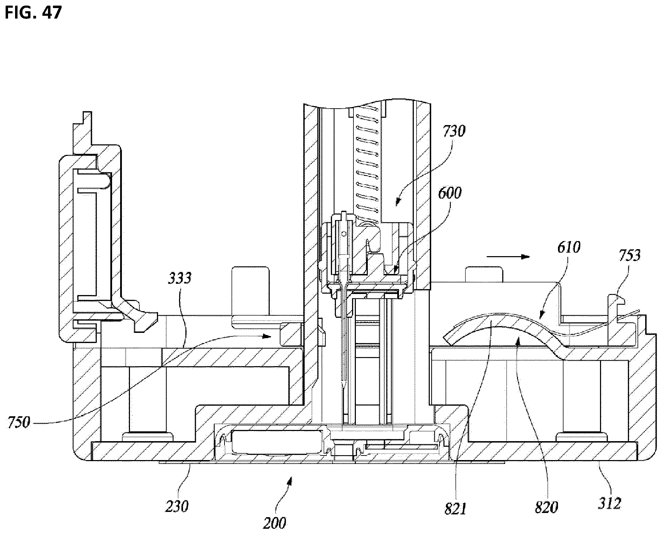

As shown in FIG. 46, when the moving member (750) is loaded in the third position, the support (820) is pressed by the moving member (750) and can be elastically deformed into a shape that does not protrude from the stage (333). And as shown in FIG. 47, when the moving member (750) moves to the fourth position and the protective sheet (610) is separated, the support (820) may deviate from the moving member (750) and protrude from the stage (333) to support the protective sheet (610) so as not to interfere with other components.

The shape or location of the support (820) can be changed in various ways.

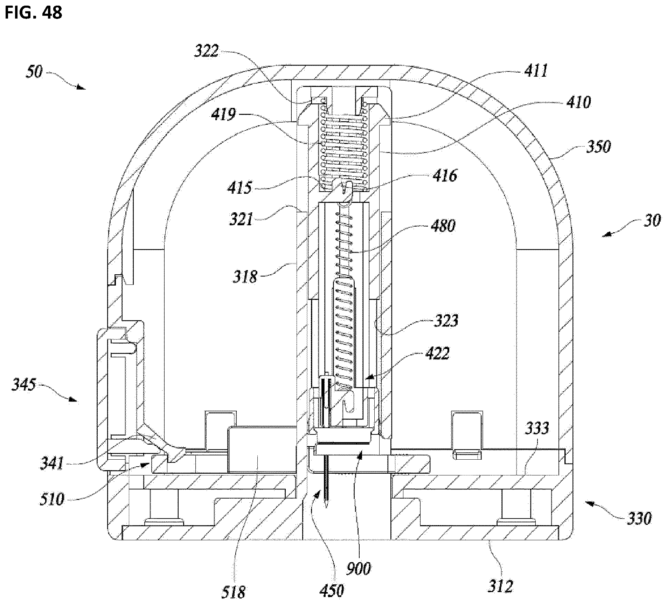

Meanwhile, FIG. 48 is a cross-sectional view showing an applicator assembly according to another embodiment of the present disclosure.

The applicator assembly (50) shown in FIG. 48 includes a sensor unit (900) having a sensor that is inserted into the skin of a user and measures biometric information, and the applicator (30) on which the sensor unit (900) is detachably mounted to insert the sensor into the skin of a user. The applicator (30) is the same as described previously.

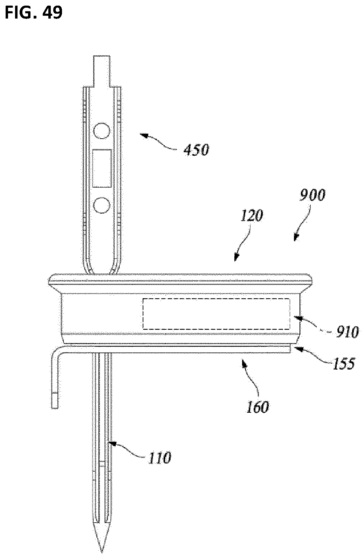

As shown in FIG. 49, the sensor unit (900) includes the sensor (110) inserted into the skin of a user, the sensor unit housing (120) to which the sensor (110) is coupled, the adhesive layer (155) provided on the sensor unit housing (120), the protective sheet (160) covering the adhesive layer (155), and an electronic component (910). The electronic component (910) includes a board electrically connected to the sensor (110), a process chip installed on the board to convert biometric information measured by the sensor (110) into an electrical signal, a communication chip for communication with the outside and batteries, etc. The sensor unit (900) can be attached to the skin of a user by moving from the first position to the second position while being coupled with the needle (450).

The applicator (30) may use the moving member (510) to remove the protective sheet (160) from the sensor unit (900) and move the sensor unit (900) from the first position to the second position.

The sensor unit (900) is inserted into the skin of a user in a second position and attached to the skin of a user by the adhesive layer (155). After the sensor unit (900) is attached to the skin of a user, the needle (450) is separated from the skin, and the sensor unit (900) is separated from the applicator (30) to measure the biometric information of a user.

The applicator assembly (50) according to this embodiment has a sensor unit (900) equipped with a signal processing function and a communication function, so that without the need for a separate base unit, the sensor unit (900) is attached to the skin of a user and measures biometric information and transmits it to an external terminal (5) or the like.

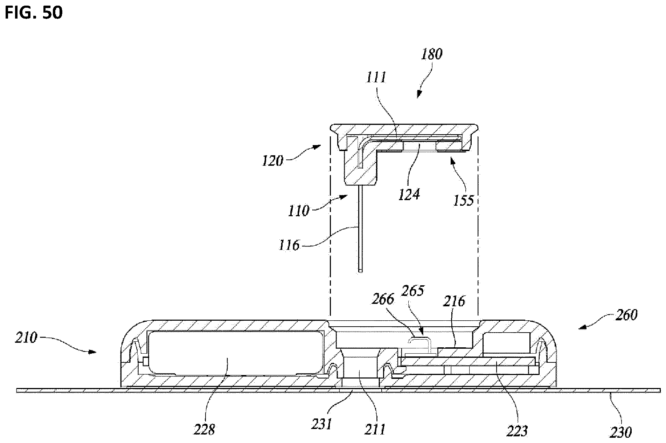

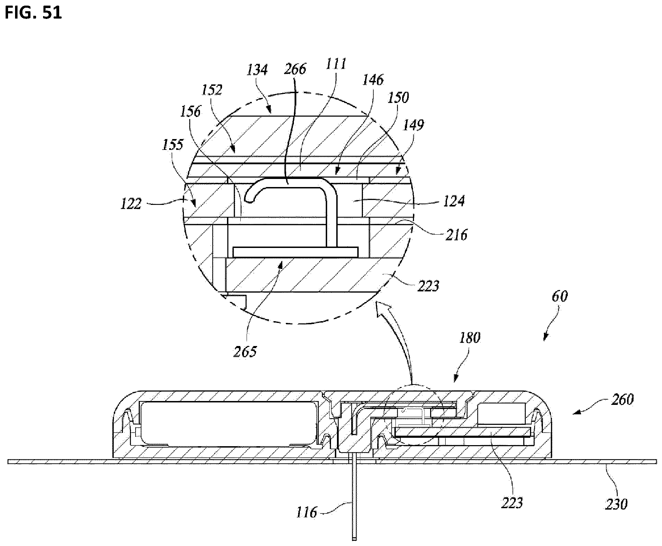

Meanwhile, FIGS. 50 and 51 show other embodiments of the sensor unit and base unit.

As shown in FIGS. 50 and 51, the sensor unit (180) includes the sensor (110) inserted into the skin of a user, the sensor unit housing (120) to which the sensor (110) is coupled, the sensor adhesive portion (149) and adhesive pad (152) for fixing the sensor (110) to the sensor unit housing (120), the adhesive layer (155) provided on the sensor unit housing (120) to be attached to the base unit (260) and the protective sheet covering the adhesive layer (155). The sensor unit (180) has a configuration in which the sensor unit-electrical contact portion for electrically connecting the sensor (110) to the base unit (260) is omitted.

The base unit (260) includes the base unit housing (210) to which the sensor unit (180) is coupled, the circuit board (223) installed inside the base unit housing (210), the base unit-electrical contact portion (265) electrically connected to the sensor (110) of the sensor unit (180) and a battery (228). Base unit-electrical contact portion (225) contact sensor (110) when sensor unit (180) is coupled to base unit (260). The base unit-electrical contact portion (225) is electrically connected to the circuit board (223), and a portion of the base unit-electrical contact portion (225) may protrude from the contact surface (216) to contact the sensor (110). The base unit-electrical contact portion (225) may include a plurality of terminal portions (266) that each contact a plurality of contact points (not shown) provided on the sensor body (111) of the sensor (110). The terminal portion (266) may electrically connect the sensor (110) and the circuit board (223) by contacting a contact point of the sensor body (111). The terminal portion (266) may be formed to be elastically deformed when in contact with the sensor body (111) so as to stably contact the sensor body (111).

The sensor unit (180) is attached to the base unit (260) by the adhesive layer (155), thereby forming a body attachable unit (60) together with the base unit (260). When the sensor unit (180) is coupled with the base unit (260), the base unit-electrical contact portion (265) passes through the adhesive layer hole (156), the through hole (124), and the sensor adhesive hole opening (150) and comes into contact with the sensor body (111) of the sensor (110). Accordingly, the sensor (110) may be electrically connected to the base unit (260) through the base unit-electrical contact portion (265). At this time, the adhesive layer (155) seals between the sensor (110) and the base unit-electrical contact portion (265) to prevent moisture or foreign substances from entering the electrical connection portion between the sensor unit (180) and the base unit (260).

In order to electrically connect the sensor (110) and the base unit (260), the specific configuration, number, and location of the base unit-electrical contacts provided in the base unit (260) may be changed in various ways.

Although the present disclosure has been described above with preferred examples, the scope of the present disclosure is not limited to the configurations described and shown above.

For example, in the drawing, the moving member for separating the protective sheet from the sensor unit supports the plunger in the third position, fixes the plunger in the first position, and moves to the fourth position to separate the protective sheet and move away from the plunger, but the applicator may have a configuration including a separate member for fixing the plunger in the first position in addition to the moving member.

Additionally, the drawing shows that the moving member is installed to move in a direction that intersects approximately perpendicular to the moving direction of the sensor unit, but the moving member may be installed to move in another direction.

Additionally, the drawing shows that the sensor unit is detachably coupled to a carrier coupled with a needle and moves from a first position to a second position, but the sensor unit is detachably coupled to the plunger or a separate member provided on the plunger and can move together with the plunger.

In addition, the drawing shows that the moving member is moved by a moving member driver, but the moving member may be moved by an operating member operated by a user.

In addition, the drawing shows that in a state in which the elastic member that provides the moving force to the moving member is elastically deformed, a stopper that restrains the movement of the moving member is provided in the middle frame, but the position of the stopper can be changed in various ways. As another exemplary embodiment, the stopper may be installed at various locations on the applicator body other than the middle frame. As another exemplary embodiment, the stopper is provided on a moving member to engage with the applicator body and can be released by the operating member.

Additionally, as another exemplary embodiment, the base unit may simply take a configuration to support the sensor unit so that it is not separated from the skin of a user. In this case, a separate electronic unit that can process biometric information measured by the sensor unit and transmit it to an external terminal may be detachably coupled to the base unit. A separate electronic unit may be coupled to the base unit to be electrically connected to the sensor unit after the sensor unit is coupled to the base unit.

Although the present disclosure has been shown and described in connection with preferred embodiments for illustrating the principles of the disclosure, the disclosure is not limited to the construction and operation as shown and described. Rather, those skilled in the art will understand that numerous changes and modifications can be made to the present disclosure without departing from the concept and scope of the attached registration claims.

## Claims

1. An applicator for inserting a sensor for measuring biometric information into skin of a user, the applicator comprising:
an applicator body configured to be contactable with the skin of the user;
an insertion unit installed to the applicator body to move a sensor unit, which comprises the sensor, a sensor unit housing to which the sensor is mounted, an adhesive layer comprised in the sensor unit housing, and a protective sheet covering the adhesive layer, from a first position where the sensor unit is spaced apart from the skin of the user to a second position where the sensor is inserted into the skin of the user;
a removing unit installed to the applicator body to separate the protective sheet from the adhesive layer before the sensor unit reaches the second position; and
an operating member installed to the applicator body such that the removing unit is operated by manipulation of the user.

2. The applicator according to claim 1,
wherein the removing unit comprises
a moving member installed movably to the applicator body to pull the protective sheet in a direction of separating the protective sheet from the adhesive layer, wherein a portion of the protective sheet is coupled to the moving member.

3. The applicator according to claim 2,
wherein the removing unit includes
a moving member driver configured to move the moving member.

4. The applicator according to claim 3,
wherein the moving member driver includes an elastic member configured to apply an elastic force to the moving member in a direction of peeling off the protective sheet from the adhesive layer.

5. The applicator according to claim 4,
comprising a stopper configured to restrain movement of the moving member in a state that the elastic member is elastically deformed,
wherein the operating member is configured to release the stopper such that the moving member is movable by the elastic member.

6. The applicator according to claim 2,
wherein the moving member is installed to move in a direction of intersecting a direction in which the sensor unit moves from the first position to the second position.

7. The applicator according to claim 6,
comprising a support installed to the applicator body to support the protective sheet separated from the adhesive layer by the moving member.

8. The applicator according to claim 7, wherein:
the moving member is installed to be movable from a third position where the protective sheet is coupled to the protective sheet in a state that the protective sheet covers the adhesive layer to a fourth position where the protective sheet is separated from the adhesive layer, and
the support is configured to be elastically deformable by being pressed by the moving member when the moving member is in the third position, and return to an original state to support the protective sheet by being released from the moving member when the moving member moves to the fourth position.

9. The applicator according to claim 2, wherein:
the insertion unit includes
a plunger installed to be movably from the first position to the second position with the sensor unit,
a needle detachably coupled to the sensor unit to be inserted into the skin of the user with the sensor,
a carrier installed to the plunger and coupled with the needle, and
a plunger driver configured to provide a moving force to the plunger in a direction of moving from the first position to the second position, and
wherein the plunger is configured such that movement of the plunger at the first position is restricted by being contacted with the moving member, and is configured to move away from the moving member toward the second position when the moving member moves in a direction of separating the protective sheet from the adhesive layer.

10. The applicator according to claim 9, wherein:
the moving member is installed to move in a direction of intersecting a direction in which the sensor unit moves from the first position to the second position, and
the plunger is configured to deviate from the moving member after a separation operation of the protective film by the moving member is completed.

11. The applicator according to claim 9, wherein:
the applicator body includes
a frame base portion configured to be contactable with the skin of the user, a base frame having a column portion protruding from the frame base portion and accommodating the plunger,
a stage disposed at an upper side of the frame base and supporting the moving member, and a middle frame having a middle frame opening formed in a middle of the stage to allow the column portion to be inserted.

12. The applicator according to claim 11, wherein:
the removing unit comprises an elastic member configured to apply an elastic force to the moving member in a direction of removing the protective sheet from the adhesive layer,
a stopper configured to restrains movement of the moving member in a state in which the elastic member is elastically deformed is comprised in one of the middle frame and the moving member, and
the operating member is configured to release the stopper such that the moving member is movable by the elastic member.

13. The applicator according to claim 9, wherein:
the moving member comprises
a moving member body,
a moving member opening formed in a middle of the moving member body to allow the plunger to pass through the moving member opening, and
a grip portion comprised in the moving member body to be coupled with the protective sheet.

14. The applicator according to claim 9, further comprising
a needle release driver configured to provide a moving force to the carrier such that the carrier moves the needle in a direction away from the skin of the user after the needle is inserted into the skin of the user with the sensor.

15. The applicator according to claim 2,
wherein the protective sheet comprises
a protective portion covering the adhesive layer, and
a wing portion extending from an edge of the protective portion to be coupled to the moving member.

16. The applicator according to claim 2, wherein:
the protective sheet has a protective sheet groove, and
the moving member includes a grip portion inserted into the protective sheet groove.

17. An applicator for inserting a sensor into skin of a user, the applicator comprising:
an applicator body to which a base unit comprising a base unit housing and an adhesive portion provided on the base unit housing to be attached to the skin of the user is separatably coupled;
an insertion unit installed to the applicator body to move the sensor unit, which comprises the sensor, a sensor unit housing to which the sensor is mounted, an adhesive layer comprised in the sensor unit housing to be adhered to the base unit housing, and a protective sheet covering the adhesive layer, from a first position where the sensor unit is spaced apart from the base unit to a second position where the sensor unit is coupled to the base unit such that the sensor is inserted into the skin of the user;
a removing unit installed to the applicator body to separate the protective sheet from the adhesive layer before the sensor unit reaches the second position; and
an operating member installed to the applicator body such that the removing unit is operated by manipulation of the user.

18. An applicator assembly comprising:
a sensor unit comprising a sensor, a sensor unit housing to which the sensor is mounted, an adhesive layer comprised in the sensor unit housing, and a protective sheet covering the adhesive layer;
an applicator body having a bottom portion configured to be contactable with skin of an user;
an insertion unit installed to the applicator body to move the sensor unit from a first position where the sensor unit is spaced apart from the skin of the user to a second position where the sensor unit is inserted into the skin of the user;
a removing unit installed to the applicator body to separate the protective sheet from the adhesive layer before the sensor unit reaches the second position; and
an operating member installed to the applicator body such that the removing unit is operated by manipulation of the user.

19. The applicator assembly according to claim 18,
further comprising a base unit comprising a base unit housing and an adhesive portion provided on the base unit housing to be attached to the skin of the user, wherein the base unit separatably coupled to the applicator body,
wherein the sensor unit housing is attached to the base unit housing by the adhesive layer at the second position.
